(19) Europäisches Patentamt / European Patent Office / Office européen des brevets

(11) **EP 4 380 583 B1**

(12) **EUROPEAN PATENT SPECIFICATION**

(45) Date of publication and mention of the grant of the patent:
**08.04.2026 Bulletin 2026/15**

(21) Application number: **22762015.0**

(22) Date of filing: **05.08.2022**

(51) International Patent Classification (IPC):
**A61K 35/34** (2015.01)   **A61P 21/00** (2006.01)

(52) Cooperative Patent Classification (CPC):
**A61K 35/34; A61P 21/00**

(86) International application number:
**PCT/EP2022/072088**

(87) International publication number:
**WO 2023/012334 (09.02.2023 Gazette 2023/06)**

(54) **MYOGENIC PROGENITOR CELLS FOR USE IN AN OPTIMIZED METHOD FOR THE PREVENTION AND TREATMENT OF ANAL INCONTINENCE**

MYOGENE VORLÄUFERZELLEN ZUR VERWENDUNG IN EINEM OPTIMIERTEN VERFAHREN ZUR VORBEUGUNG UND BEHANDLUNG VON ANALINKONTINENZ

CELLULES PROGÉNITRICES MYOGÉNIQUES DESTINÉES À ÊTRE UTILISÉES DANS UN PROCÉDÉ OPTIMISÉ POUR LA PRÉVENTION ET LE TRAITEMENT DE L'INCONTINENCE ANALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.08.2021 EP 21190102**

(43) Date of publication of application:
**12.06.2024 Bulletin 2024/24**

(73) Proprietor: **Innovacell GmbH**
**6020 Innsbruck (AT)**

(72) Inventors:
• **THURNER, Marco**
  **6020 Innsbruck (AT)**
• **MARKSTEINER, Rainer**
  **6130 Schwaz (AT)**

(74) Representative: **Drexl, Janna**
**Dehmel & Bettenhausen**
**Patentanwälte PartmbB**
**Herzogspitalstraße 11**
**80331 München (DE)**

(56) References cited:
• THURNER MARCO ET AL: "Generation of myogenic progenitor cell-derived smooth muscle cells for sphincter regeneration", vol. 11, no. 1, 1 December 2020 (2020-12-01), XP055881356, Retrieved from the Internet <URL:https://stemcellres.biomedcentral.com/track/pdf/10.1186/s13287-020-01749-w.pdf> DOI: 10.1186/s13287-020-01749-w
• ROMANISZYN M ET AL: "Implantation of autologous muscle-derived stem cells in treatment of fecal incontinence: results of an experimental pilot study", TECHNIQUES IN COLOPROCTOLOGY, SPRINGER-VERLAG ITALIA, MILAN, IT, vol. 19, no. 11, 13 August 2015 (2015-08-13), pages 685 - 696, XP035894153, ISSN: 1123-6337, [retrieved on 20150813], DOI: 10.1007/S10151-015-1351-0
• FRUDINGER A. ET AL: "Autologous skeletal-muscle-derived cell injection for anal incontinence due to obstetric trauma: a 5-year follow-up of an initial study of 10 patients", COLORECTAL DISEASE, vol. 17, no. 9, 1 September 2015 (2015-09-01), GB, pages 794 - 801, XP055881304, ISSN: 1462-8910, DOI: 10.1111/codi.12947

**Description**

**FIELD OF THE INVENTION**

The present invention relates to myogenic progenitor cells (MPCs), namely myoblasts, for use in a method for the treatment of anal incontinence in a subject, wherein the myoblasts are administered by one or more injections into and/or adjacent to the anal sphincter apparatus of said subject, wherein said subject suffers from anal incontinence for 6 months to 10 years and the subject has a severity of incontinence defined as more than 2 weekly incontinence episodes prior to treatment, wherein said incontinence episodes result in that liquid or solid feces unexpectedly leaked from the rectum of a subject, and wherein incontinence episodes that only appear as traces in form of staining or marks in the linen are excluded. Said myoblasts may be comprised in a pharmaceutical composition comprising a pharmaceutical acceptable excipient and/or carrier.

**BACKGROUND OF THE INVENTION**

Anal incontinence and its epidemiology

The ability to maintain continence is fundamental for our well-being as social beings. The loss of anal continence results in physical, physiological, and social handicaps. Generally, it is thought, that primarily elderly and handicapped people suffer from anal incontinence, however, these symptoms can occur in people of every age. The spectrum of anal incontinence, i.e., the loss of control of the content of the intestine, ranges from minor feces marks (traces) in the underwear to the loss of flatus up to massive episodes of uncontrolled defecation of soft or solid feces. The reasons for this can be multilayered and complex. Independently of the extremely impaired life quality for the affected individual, impaired anal continence results in a not to be underestimated cost factor for the public health system. In the USA more than 400 million dollars are spent per year for anal incontinence help programs. Furthermore, anal incontinence is the second most frequent reason for hospitalization in nursery homes (more frequently than dementia). One third of the elderly people in nursery homes or hospitals are incontinent for feces.

Anatomy and physiology of anal continence

Anatomic structures necessary for anal continence were studied in more detail in the last decade due to the possibility of endoanal imaging techniques. This also let to an improved understanding of the anal continence mechanism and the factors responsible for maintaining anal continence. Anal continence requires the coordination of different anatomic structures with different physiological functions. An intact sensibility ensures the perception of the status of rectal filling and recognition of the quality of feces. A functional innervation enables the specialized ring-shaped muscles (sphincters) to respond to an increased anal "closing request" in an appropriate manner (voluntarily and involuntarily). Finally, if the sphincter muscles are intact, they provide for the complete occlusion of the anal canal until defecation is appropriate. The dysfunction of one of these structures results in a disturbance of anal continence. Functionality of the sphincter apparatus is based on the involuntary resting pressure of the Musculus sphincter ani internus (internal anal sphincter), consisting of smooth muscle tissue, and the involuntary resting pressure and voluntary squeeze pressures originating from the Musculus sphincter ani externus (external anal sphincter). The constant base line tonus of the M. puborectalis results in a "contortion" of the anorectal transition towards the symphysis forming an angle of 90° between the anal canal and the rectum. This anorectal angle also contributes to the maintenance of anal continence. However, maintaining continence is not possible by the M. puborectalis alone. The anal continence is further provided by an interaction of the internal and external anal sphincters. The hemorrhoidal cushions of the anal canal mucosa squeezed together by the sphincter muscles ultimately provide for airtight occlusion. In the resting state the anal canal is occluded by the constant tonic activity of the external anal sphincters and the base line resting pressure of the internal anal sphincter. The internal anal sphincter, which is a continuation and enlargement of the circular smooth muscle layer of the colon, provides for about 75-85% of the base line pressure of the closed anal canal. The activity of this smooth muscle component is completely inhibited by a rectal distension, the so called rectal anal inhibitory reflex. This relaxation is accompanied by a reflex contraction of the external anal sphincter and M. puborectalis to prevent defecation if inappropriate. If defecation at that time is convenient, the external anal sphincter and puborectalis muscle relax, leading to an involuntary colorectal motor activity resulting in a greater rectal pressure than anal pressure followed by fecal expulsion. If the defecation is inconvenient at the time of occurring urge to defecate, defecation can be deferred by voluntary contraction of the puborectalis and external anal sphincter muscle thereby pushing back the feces into rectum until defecation is convenient.

Cause of anal incontinence

The importance of the described structures in terms of continence is highlighted as dysfunction of these structures leads to incontinence. Most patients suffering from anal incontinence are diagnosed with abnormalities in their anal sphincter muscles. These abnormalities can occur in the external anal sphincter, the internal anal sphincter or both. Sphincter related incontinence may be caused by (1) obstetric or accidental trauma, (2) iatrogenic trauma such as surgery or radiation, (3) neurogenic diseases such as multiple sclerosis or diabetes mellitus or (4) muscle atrophy associated with aging. Obstetric trauma is the main cause of incontinence in women since up to 9 % of vaginal deliveries lead to rupture of the anal sphincter.

Treatment of anal incontinence

When conservative treatments such as dietary changes, biofeedback exercises and antidiarrhea medication fail, lead to insufficient treatment success or were not possible to perform in the first place due to severe anatomic physiologic or neurologic dysfunction, patients are referred to invasive treatments. Traditional invasive treatments include surgical approaches such as anal sphincter repairs and recreations (sphincteroplasty, artificial anal sphincter, graciloplasty), if the sphincter muscles are defect, or colostomy and antegrade continence enema, if incontinence occurs due to complete sinal cord impairment. However, traditional surgical approaches for sphincter repair have failure rates as high as 50 % and surgical approaches to create a new sphincter have high morbidity rates. One less invasive treatment for incontinence is the perianal injection of bulking agents, which is supposed to augment anal canal pressures. A single randomized sham-controlled trial found that injection of NASHA Dx (a dextranomer stabilized in hyaluronic acid) led to a significantly better treatment outcome than sham injection. In detail, a reduction of at least 50 % incontinence episodes per week in 52 % of patients compared to 32 % of patients that underwent sham treatment was found. However, only 6 % of patients were found completely continent and efficacy data available to date only covers short term effects of up to 6 months. Also limited effects of bulking agent on patient quality of life was noted. Thus, more effective treatments for anal incontinence are needed. During the last two decades neuromodulation raised increasing attention as a treatment option for incontinence, of which sacral nerve stimulation (SNS) became the most promising in terms of effectivity. SNS therapy is based on the implantation of a medical device (e.g. Interstim, Medtronic, USA), employing chronic low-voltage electrical stimulation on the sacral nerve to improve neuromuscular function, however with yet unclear mode of action (MoA). Success rates, defined as percent of patients with at least 50 % reduction in weekly incontinence frequency (IEF) following SNS, were summarized from 61 trials and a median success rate for short term (≤12 months), medium term (12-36 months) and long term (>36 months) of 63, 58 and 54%, respectively, was identified. However, 13.1 % of patients screened did not respond to test stimulation and thus did not receive permanent implantation, and about 25 % of patients that were included into long term follow up had explanation of the stimulation device due to complications (technical or infections) or loss of efficacy.

As incontinence is mostly caused by deficiencies/weakness of the musculature of the rectum, animal models of muscle regeneration suggested that intramuscularly injected cells engraft within host muscle and early clinical trials on treatment of stress urinary incontinence by injection of muscle derived cells were promising, the idea of regenerating the anal sphincter by cell therapy was born (Frudinger et al., 2009). However, to allow market approval and thus broad application of cell therapy for incontinence treatment, efficacy of the treatment methods needs to be superior to placebo effects and needs to be clinically relevant in that the subjects in need have sufficient benefit and/or complete remission. An effect of reduction of at least 50% of the weekly incontinence episodes is considered as clinically relevant. For a method to be useful a significantly higher proportion of cell therapy treated patients compared to placebo treated patients needs to have such a reduction to consider a method effective.

As outlined above, incontinence is a highly complex condition with many different causes, histories, and severities, thus selection of the most appropriate patient population which can benefit from a specific method is required. For each treatment method, a different population of patients might be the target to achieve significant and relevant treatment effects.

EP210976B1 discloses methods for use of myoblasts for the prevention and treatment of anal incontinence by injecting said cells into the external anal sphincter, wherein said subject has suffered a rectal injury. WO2014/044867A1 discloses a treatment method for women and men with urinary and/or anal incontinence or in risk of developing urinary or anal incontinence by administration of skeletal muscle derived cells. WO2019/115790 discloses skeletal muscle-derived cells for use in the treatment of incontinence. WO2020/193460 discloses induced smooth muscle cells for use in a method of treating a disease or disorder in a subject. Clinical studies were conducted to test the potential efficacy of muscle cells for treatment of fecal incontinence, however, did not demonstrate superior effects of the method compared to placebo effects. Also, Thurner et al. (Stem Cell Research & Therapy, vol. 11, no 1, 2020), Romaniszyn et al. (Techniques in Coloproctology, Springer-Verlag Italia, Milan, IT, vol. 19, no. 11, 2015, pages 685-696) and Frudinger et al. (Colorectal Disease, vol. 17, no. 9, 2015, pages 794-801) disclose the local injection of myoblasts in the sphincter muscle for use in treating anal/fecal incontinence.

## SUMMARY OF THE INVENTION

The invention is defined by the appended claims. The present invention was made in light of the prior art described above. Thus, one object of the present invention is the provision of an effective method for use of myogenic progenitor cells for the prevention and/or treatment of anal incontinence.

The inventors have surprisingly found that while in some subjects, injections of myogenic progenitor cells had no or only little effects, injections of myogenic progenitor cells resulted in other subjects in a significant improvement of the subject's condition.

None of the above-mentioned disclosures selects a narrowly defined patient population within the broad indication of incontinence ultimately eligible to be administered with myogenic progenitor cells for improved prevention/-treatment of anal incontinence. Thus, it was an object underlying the present invention to identify the group of subjects which can effectively be treated by injections of myogenic progenitor cells in the context of a personalized treatment approach. Another object of the present invention is the provision of an optimized method for use of myogenic progenitor cells for the prevention and/or treatment of anal incontinence, which method results in a more effective treatment of anal incontinence.

None of the above-mentioned disclosures selects a patient population according to subjects' conditions associated with incontinence, duration of incontinence and/or severity of incontinence. Thus, another object of the present invention is the provision of selecting patient populations according to subjects' conditions associated with incontinence, duration of incontinence and/or severity of incontinence in a method for use of myogenic progenitor cells for the prevention and/or treatment of anal incontinence.

None of the above-mentioned disclosures selects a safe and effective number of cells for prevention and/or treatment of anal incontinence. Thus, one object of the present invention is the selection of a specific cell dose in a method for use of myogenic progenitor cells for the prevention and/or treatment of anal incontinence.

Further, none of the above-mentioned disclosures selects optimized stimulation concomitant in a method for use of myogenic progenitor cells for the prevention and/or treatment of anal incontinence. Thus, another object of the present invention is the selection of optimized concomitant stimulation in a method for use of myogenic progenitor cells for the prevention and/or treatment of anal incontinence.

The objects of the present invention are solved by the subject-matter defined in the claims.

## BRIEF DESCRIPTION OF DRAWINGS

The following figures form part of the present specification and are included to further demonstrate certain aspects of the present invention. The invention may be better understood by reference to one or more of these drawings in combination with the detailed description of specific embodiments presented herein.

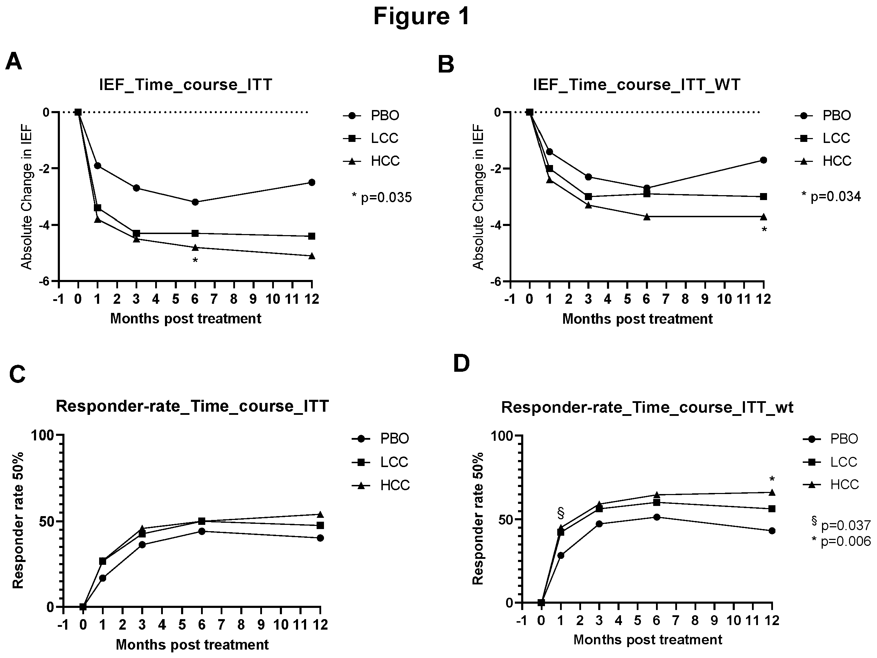

Figure 1 illustrates the changes in weekly incontinence episode frequency (IEF) from baseline (A, B) as well as 50% responder rates (C, D) over the period of the study of Example 5 among treatment groups (Low cell count - LCC, High Cell Count - HCC, Placebo - PBO) in the intention to treat (ITT) population, wherein episodes classified as traces were counted (A, C) or excluded (B, D) for analyses.

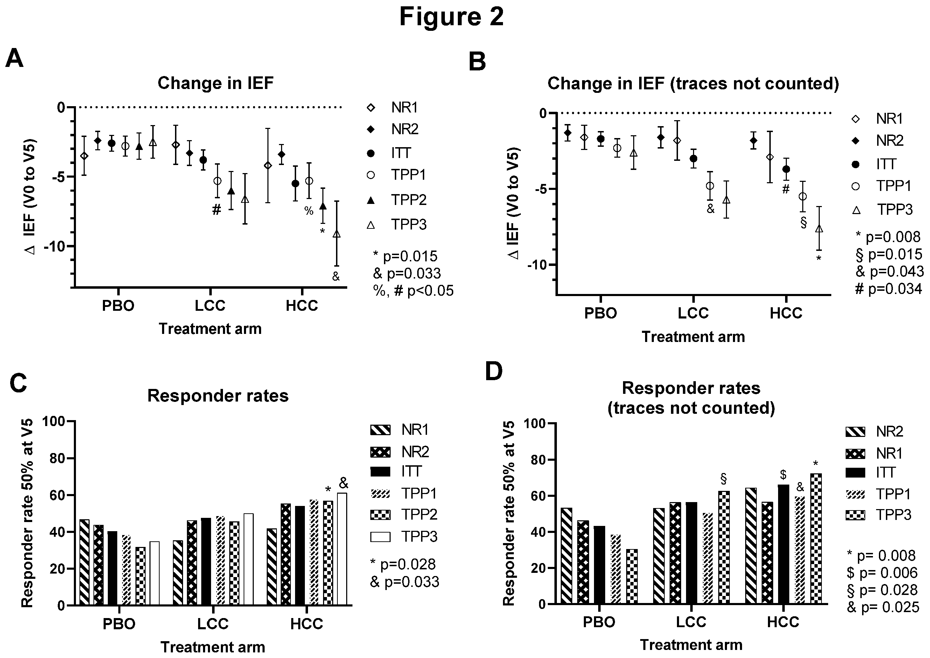

Figure 2 illustrates change in weekly IEF from baseline to 12 months post treatment (A, B) and 50% responder rates at 12 months post treatment (C, D) according to Example 5 each either calculated by including (A, C) or excluding (B, D) traces from being counted as incontinence episodes. Data visualized among treatment groups (PBO, LCC, HCC) in different patient populations according to subject selection of Example 6. Different patient populations defined in Example 6 referred to as TPP1 are patients suffering from fecal incontinence for 10 or less than 10 years, TPP2 are patients suffering from more than 2 episodes at baseline classified as little or more, TPP3 are patients suffering from fecal incontinence for 10 or less than 10 years and more than 2 weekly IEF at baseline classified as little or more. NR1 are patients that suffer from fecal incontinence (FI) for more than 10 years and NR2 are patients that suffered from 2 or less than 2 incontinence episodes (IE) classified as "little" or "more". Whenever p-values of two-sided Wilcoxon tests (A, B) or Fisher's Exact tests (C, D) comparing PBO with LCC or HCC reached significance threshold of $p < 0.05$, p-values are shown.

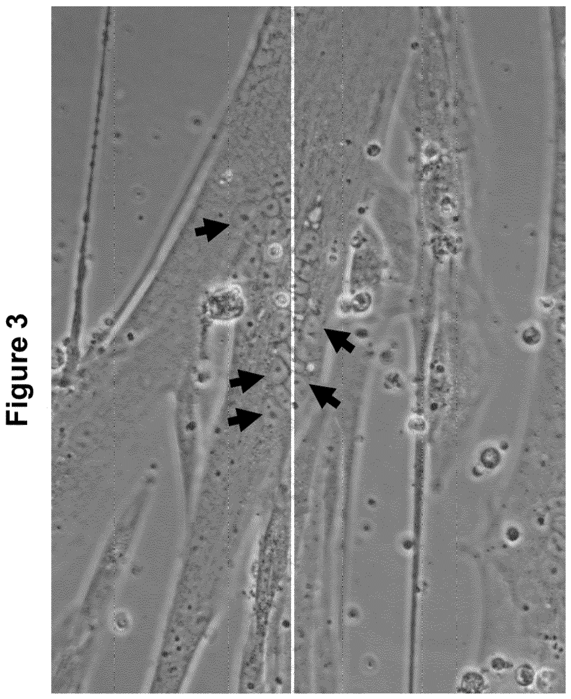

Figure 3 illustrates a phase contrast microscopic image of myogenic progenitor cells isolated by Example 1 and tested for differentiation potential according to Example 2. A myotube is visible at a magnification of 600x containing multiple nuclei (black arrows), thereby demonstrating the potential of single nucleated MPCs to fuse with each other.

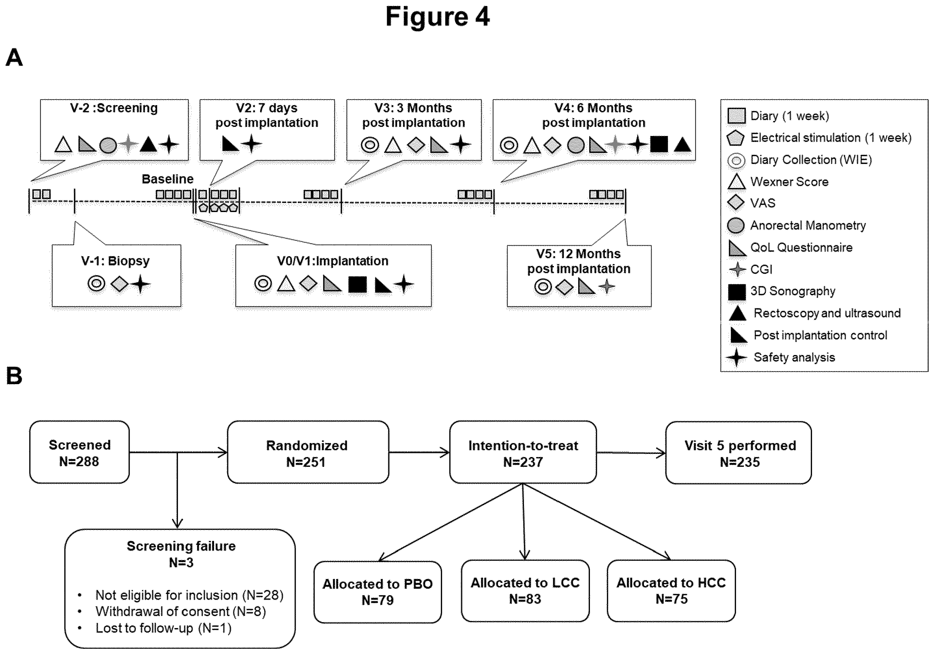

Figure 4 illustrates the clinical study overview conducted according to Example 5. Study visits, including parameters evaluated, and timeline for data collection are shown in A. Screened patients, screening failures, randomized patients as well as intention to treat (ITT) patients according to their treatment allocation are shown in numbers (N) in B. QoL = quality of life, V = visit, VAS = visual analogue scale, WIE = weekly incontinence episodes, CGI = Clinical global impression scale.

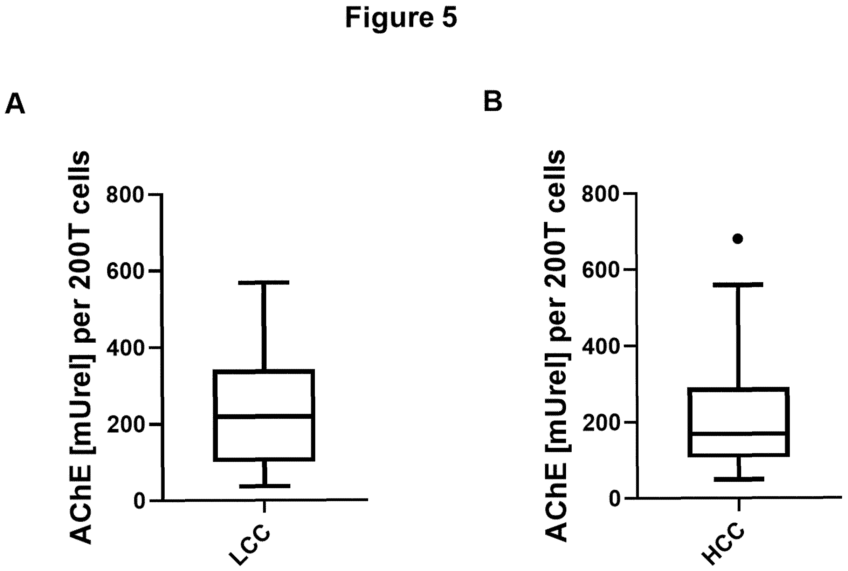

Figure 5 illustrates Tukey's box plots summarizing the Acetylcholinesterase (AChE) activity of LCC (A) and HCC (B) batches containing MPCs isolated according to Example 1 and measured for AChE activity according to Example 3 following *in vitro* differentiation according to Example 2. AChE activity is demonstrated as relative mU per $2*10^5$ cells. LCC batches (n=83) were found to harbor a mean±SD AChE activity of 241.90±151.90 ranging between 36 and 568. HCC batches (n=75) were found to harbor a mean±SD AChE activity of 213±137.40 ranging between 49 and 680.

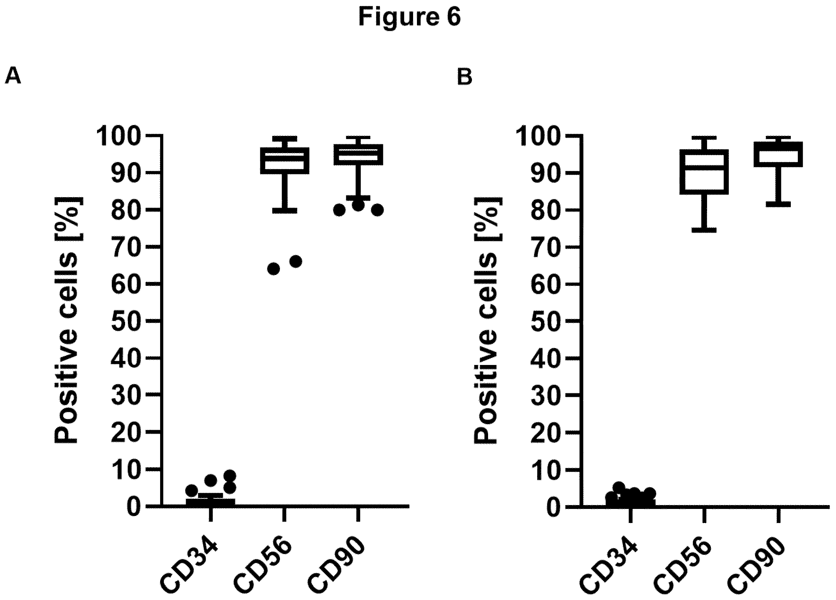

Figure 6 illustrates percentage of MPCs positive for surface cell marker CD34, CD56 and CD90 of either low cell count (LCC, n=83) (A) and high cell count (HCC, n=75) formulations prepared according to Example 1 and tested for surface marker according to Example 4. Data visualized as Tukey's bar graphs (outlier visible as dots). All LCC and HCC batches were found to be CD34 negative (% CD34 positive at a mean±SD of 1.29±1.37 for LCC and 1.10±0.90 for HCC). All LCC and HCC batches were found to be CD56 positive (% CD56 positive at a mean±SD of 92.06±6.73 for LCC and 89.86±7.06 for HCC). All LCC and HCC batches were found to be CD90 positive (% CD90 positive at a mean ±SD of 94.00±5.00 for LCC and 94.99±4.52 % for HCC).

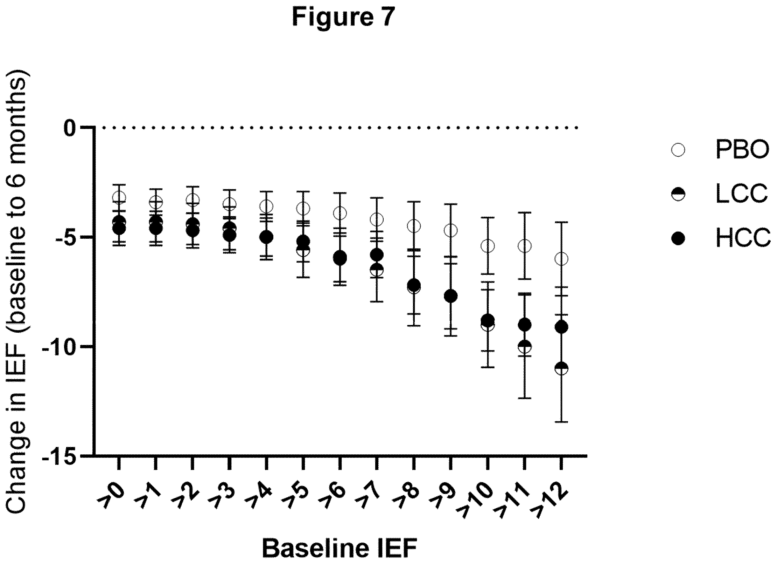

Figure 7 illustrates change in IEF from baseline to 6 months post treatment among treatment groups (PBO, LCC, HCC) in ITT set patients further subgrouped according to increasing number of baseline IEF (including traces). Data visualized as mean±SEM. IEF changes from baseline to 6 months increased by continuous exclusion of lower baseline IEF patients in all groups but more prominently in LCC and HCC than PBO patients.

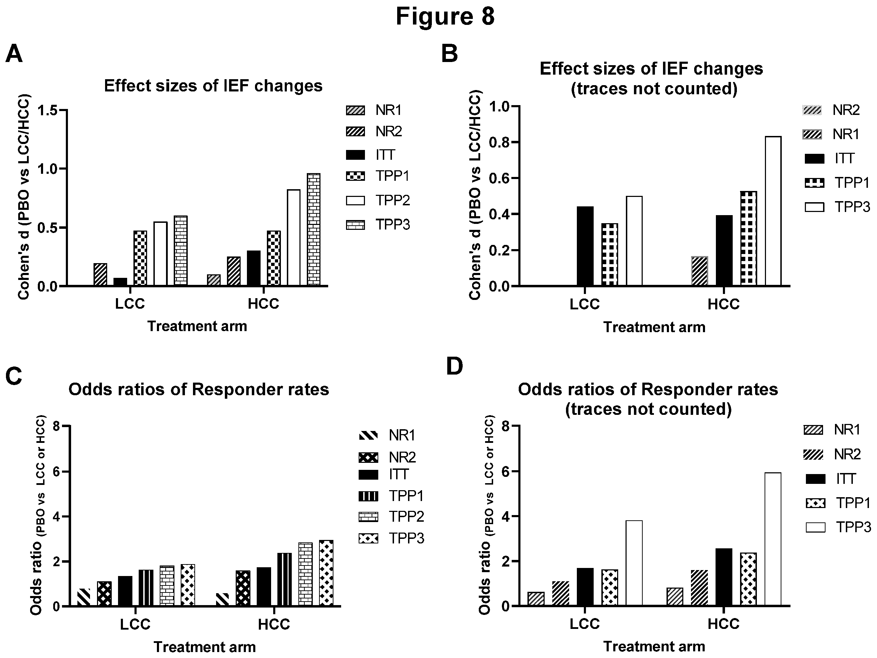

Figure 8 illustrates effect sizes (Cohen's d) of IEF from baseline to 12 months post treatment (A, B) as well as Odds ratios of 50% responder rates at 12 months post treatment (C, D), each calculated by comparing LCC or HCC with PBO treatment either considering traces as incontinence episodes (A, C) or not (B, D) in ITT, TPP1, TPP2, TPP3, NR1 and NR2 patient populations.

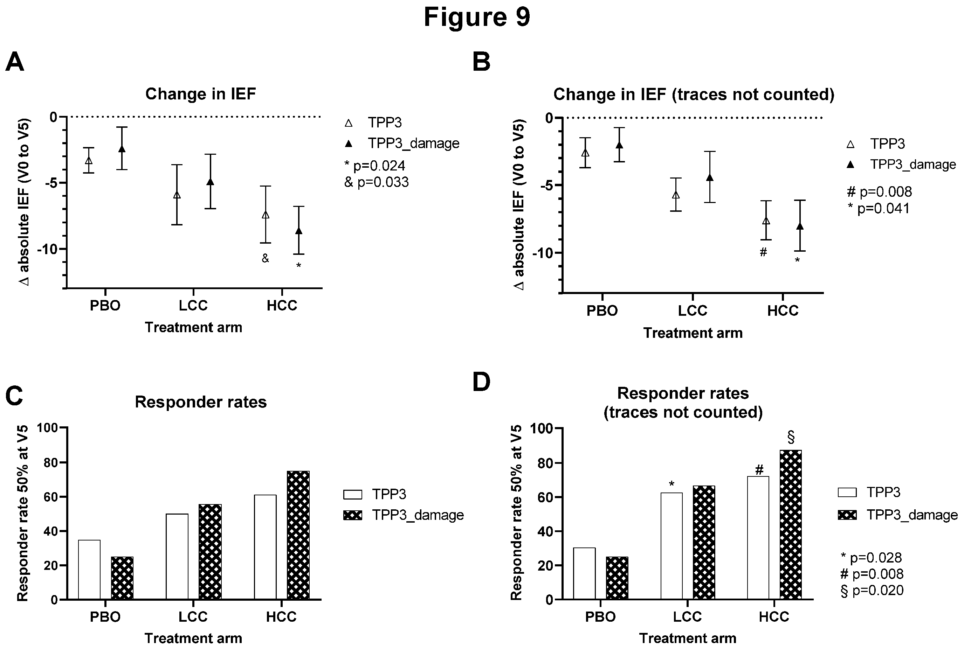

Figure 9 illustrates change in IEF from baseline to 12 months post treatment (A, B) and 50% responder rates (C, D) among treatment groups (PBO, LCC, HCC) in TPP3 set patients and TPP3 patients further subgrouped to select only patients having fecal incontinence associated with anal sphincter muscle damage (TPP3_damae). Data either shown including traces as incontinence episodes (A, C) or excluding them (B, D). Data visualized as mean±SEM. P-values of two-sided Wilcoxon-Rank-sum tests comparing LCC or HCC with PBO and being below alpha error of 0.05 are demonstrated for IEF changes. P-values of Fisher's exact test comparing responder rates of LCC or HCC with PBO treatment are shown if below 0.05.

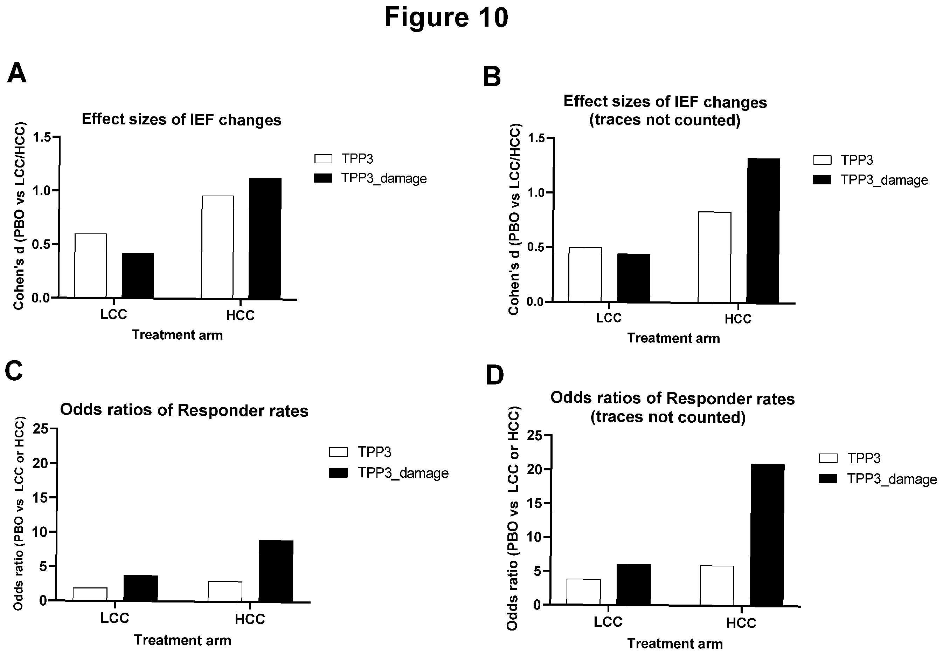

Figure 10 illustrates effect sizes (Cohen's d) of IEF from baseline to 12 months post treatment (A, B) as well as Odds ratios of 50% responder rates at 12 months post treatment (C, D), each calculated by comparing LCC or HCC with PBO treatment either considering traces as incontinence episodes (A, C) or not (B, D) in TPP3 and TPP3_damage patient populations.

**TERMS AND DEFINITIONS**

The term "anal incontinence," as used herein, refers to any undesired loss of intestine content through the anus, like flatus, liquid or solid faeces. The term comprises all three severity grades: Grade 1 = only gaseous, grade 2 = liquid and soft feces, grade 3 = solid, formed feces.

The term "anal sphincter" or "anal sphincter apparatus," as used herein, refers preferably to the *Musculus sphincter ani externus* and the *Musculus puborectalis* as a part of the *Musculus levator ani* and the *Musculus sphincter ani internus.* However, it may also include *M. pubococcygeus, M. ischiococcygeus, M. iliococcygeus* and *N. pudendus.*

The term "fecal incontinence" as used herein, refers preferably to any undesired loss of intestine content through the anus restricted to liquid or solid feces. The term may comprise the "anal incontinence" severity grades: Grade 2 = liquid and soft feces, Grade 3 = solid, formed feces.

The term "myogenic progenitor cells" or in short "MPCs" as used herein preferably refers to cells with myogenic potential, which can be primary cells and/or *in vitro* cultured cells derived from muscle tissue and alternatively relates to other cells with myogenic potential derived e.g. from adipose tissue or other stem cell harboring tissue such as but not limited to bone marrow. The term also comprises cells that are pluripotent stem cells or cells derived from pluripotent stem cells that can be isolated and cultured to become muscle cells or become muscle cells following administration into or adjacent to a muscle tissue. As myogenic progenitor cells are preferably obtained by isolation of muscle tissue, the term "myogenic progenitor cells" may also refer to a suspension of several distinct cell types wherein at least 50%, 60%, 70%, 80%, 90%, 95%, 98% or at least 99% of said cells are "myogenic progenitor cells" as defined herein. Preferably, said suspension is a suspension of single cells, wherein at least 50%, 60%, 70%, 80%, 90%, 95%, 98% or at least 99% of said single cells are homogeneous of the same cell type, namely myogenic progenitor cells. Besides the myogenic progenitor cells as defined herein, said suspension may comprise also other cell types such as cells of adipose-, chondrogenic-, osteogenic- and/or fibrogenic- tissue of origin. Such cells may be mesenchymal cells, adipocytes, chondrocytes, osteocytes and/or any other cell type that may be a process related impurity and/or may be isolated alongside the myogenic progenitor cells. Such cells may comprise a maximum of 1%, 2%, 5%, 10%, 20%, 30%, 40%, or 50% of the myogenic progenitor cell suspension.

The term "myogenic potential" refers preferably to the possibility of a cell, cells, or a cell population to develop, regenerate and/or augment muscle tissue. Myogenic potential as used herein may further relate to the potential of a cell, cells or a cell population to *in vitro* differentiate to multinucleated myotubes and/or express enzymatically active Acetylcholinesterase.

The term "penetration," as used herein, preferably refers to a process of introducing an injection device, for instance a needle into a body tissue without effecting the injection process yet.

The term "injection" as used herein, preferably refers to the expulsion of an injection solution releasing above mentioned cells out of an injection device into a specific site within the human body, in particular into or adjacent to muscle-tissue providing for anal continence (e.g. anal sphincter apparatus). The injection process may be, but is not limited to, a static injection, i.e., the injection device remains at the position reached. Said static injection process preferably refers to an injection process, wherein during expulsion of an injection solution, said injection device is static, i.e. not moving relative to the expulsed injection solution. Alternatively, the injection process may be dynamic. Said dynamic injection process preferably refers to an injection process, wherein during expulsion of an injection solution, said injection device is dynamic, i.e. moving relative to the expulsed injection solution. A dynamic injection process may for example be conducted by moving a hollow needle, which is placed e.g. within the external anal sphincter, backwards through the trajectory of the needle and thereby simultaneously expulsing an injection solution. Alternatively preferred, the term "injection" as used herein, refers to any route of administration suitable to bring cells adjacent to the anal sphincter apparatus. Such routes of administration, preferably comprise oral-, topical-, intravenous- or intraarterial administration.

The term "injection site" as used herein, preferably refers to a site within the human body, such as but not limited to a site close to or being muscle-tissue providing for anal continence, at which the injection process may be initiated. The injection site may be identical or it may not be identical with the site where the injection process ends.

The term "injection device" as used herein, comprises any device suitable for penetrating human tissue in order to reach an injection site of interest and to deliver solutions to said injection side, in particular solutions comprising myogenic progenitor cells to the injection site of interest.

The term "passive incontinence" as used herein, preferably refers to a lack of sensory recognition of loss of feces. This comprises low anal base line pressure values and a lacking sensory ability of the anal and rectal mucosa.

The term "urge incontinence" or "imperative urgency" as used herein, preferably refers to the lacking ability of a person to delay defecation for more than five minutes following recognition of an urge to defecate. Such a patient must go to the toilette immediately and/or cannot reach the toilet soon enough to deposit intestinal content into a toilette resulting in involuntary defecation.

The term "CD56+" or "CD56 positive" as used herein preferably refers to a cell expressing the cell marker CD56. The terms "CD56+" or "CD56 positive" may also be used for a cell population comprising different cell types, if preferably at least 50, 60, 70, 80, 90, 95, 98 or 99 percent of the cell population express the cell marker CD56.

The term "CD56-" or "CD56 negative" as used herein preferably refers to a cell not expressing the cell marker CD56. The terms "CD56-" or "CD56 negative" can also be used for a cell population comprising different cell types, if preferably at most 49, 40, 30, 20, 10, 5, 4, 3, 2, 1 or 0 percent of the cell population express the cell marker CD56.

The term "A2B5+" or "A2B5 positive" as used herein preferably refers to a cell expressing the cell marker A2B5. The terms "A2B5+" or "A2B5 positive" may also be used for a cell population comprising different cell types, if preferably at least 50, 60, 70, 80, 90, 95, 98 or 99 percent of the cell population express the cell marker A2B5.

The term "A2B5-" or "A2B5 negative" as used herein preferably refers to a cell not expressing the cell marker A2B5. The terms "A2B5-" or "A2B5 negative" may also be used for a cell population comprising different cell types, if preferably at most 49, 40, 30, 20, 10, 5, 4, 3, 2, 1 or 0 percent of the cell population express the cell marker A2B5.

The term "desmin positive" as used herein preferably refers to a cell expressing the cell marker desmin. The term "desmin positive" may also be used for a cell population comprising different cell types, if preferably at least 50, 60, 70, 80, 90, 95, 98 or 99 percent of the cell population express the cell marker desmin.

The term "desmin negative" as used herein preferably refers to a cell not expressing the cell marker desmin. The term "desmin negative" may also be used for a cell population comprising different cell types, if preferably at most 49, 40, 30, 20, 10, 5, 4, 3, 2, 1 or 0 percent of the cell population express the cell marker desmin.

The term "CD105+" or "CD105 positive" as used herein preferably refers to a cell expressing the cell marker CD105. The terms "CD105+" or "CD105 positive" may also be used for a cell population comprising different cell types, if preferably at least 50, 60, 70, 80, 90, 95, 98 or 99 percent of the cell population express the cell marker CD105.

The term "CD105-" or "CD105 negative" as used herein preferably refers to a cell not expressing the cell marker CD105. The terms "CD105-" or "CD105 negative" may also be used for a cell population comprising different cell types, if preferably at most 49, 40, 30, 20, 10, 5, 4, 3, 2, 1 or 0 percent of the cell population express the cell marker CD105.

The term "CD34+" or "CD34 positive" as used herein preferably refers to a cell expressing the cell marker CD34. The terms "CD34+" or "CD34 positive" can also be used for a cell population comprising different cell types, if preferably at least 50, 60, 70, 80, 90, 95, 98 or 99 percent of the cell population express the cell marker CD34.

The term "CD34-" or "CD34 negative" as used herein preferably refers to a cell not expressing the cell marker CD34. The terms "CD34-" or "CD34 negative" can also be used for a cell population comprising different cell types, if preferably less than 50% or at most 49, 40, 30, 20, 10, 5, 4, 3, 2, 1 or 0 percent of the cell population express the cell marker CD34. In a particularly preferred embodiment, the term "CD34-" or "CD34 negative" can be also used for a cell population comprising different cells, if preferably at most 19, 10, 5, 4, 3, 2, 1 or 0 percent of the cell population express the cell marker CD34.

The term "CD90+" or "CD90 positive" as used herein preferably refers to a cell expressing the cell marker CD90. The terms "CD90+" or "CD90 positive" can also be used for a cell population comprising different cell types, if preferably at least 50, 60, 70, 80, 90, 95, 98 or 99 percent of the cell population express the cell marker CD90.

The term "CD90-" or "CD90 negative" as used herein preferably refers to a cell not expressing the cell marker CD90. The terms "CD90-" or "CD90 negative" can also be used for a cell population comprising different cell types, if preferably less than 50% or at most 49, 40, 30, 20, 10, 5, 4, 3, 2, 1 or 0 percent of the cell population express the cell marker CD90.

The term "Tuj1+" or "Tuj1 positive" as used herein preferably refers to a cell expressing the cell marker Tuj1. The terms "Tuj1+'' or "Tuj1 positive" can also be used for a cell population comprising different cell types, if preferably at least 50, 60, 70, 80, 90, 95, 98 or 99 percent of the cell population express the cell marker Tuj1.

The term "Tuj1-" or "Tuj1 negative" as used herein preferably refers to a cell not expressing the cell marker Tuj1. The terms "Tuj1-" or "Tuj1 negative" can also be used for a cell population comprising different cell types, if preferably less than 50% or at most 49, 40, 30, 20, 10, 5, 4, 3, 2, 1 or 0 percent of the cell population express the cell marker Tuj1.

The term "Nestin+" or "Nestin positive" as used herein preferably refers to a cell expressing the cell marker Nestin. The terms "Nestin+" or "Nestin positive" can also be used for a cell population comprising different cell types, if preferably at least 50, 60, 70, 80, 90, 95, 98 or 99 percent of the cell population express the cell marker Nestin.

The term "Nestin-" or "Nestin negative" as used herein preferably refers to a cell not expressing the cell marker Nestin. The terms "Nestin-" or "Nestin negative" can also be used for a cell population comprising different cell types, if preferably less than 50% or at most 49, 40, 30, 20, 10, 5, 4, 3, 2, 1 or 0 percent of the cell population express the cell marker Nestin.

The term "Sca-1+" or "Sca-1 positive" as used herein preferably refers to a cell expressing the cell marker Sca-1. The terms "Sca-1+" or "Sca-1 positive" can also be used for a cell population comprising different cell types, if preferably at least 50, 60, 70, 80, 90, 95, 98 or 99 percent of the cell population express the cell marker Sca-1.

The term "Sca-1-" or "Sca-1 negative" as used herein preferably refers to a cell not expressing the cell marker Sca-1. The terms "Sca-1-" or "Sca-1 negative" can also be used for a cell population comprising different cell types, if preferably less than 50% or at most 49, 40, 30, 20, 10, 5, 4, 3, 2, 1 or 0 percent of the cell population express the cell marker Sca-1.

The term "MyoD +" or "MyoD positive" as used herein preferably refers to a cell expressing the cell marker MyoD. The terms "MyoD +" or "MyoD positive" can also be used for a cell population comprising different cell types, if preferably at least 50, 60, 70, 80, 90, 95, 98 or 99 percent of the cell population express the cell marker MyoD.

The term "MyoD -" or "MyoD negative" as used herein preferably refers to a cell not expressing the cell marker MyoD. The terms "MyoD -" or "MyoD negative" can also be used for a cell population comprising different cell types, if preferably less than 50% or at most 49, 40, 30, 20, 10, 5, 4, 3, 2, 1 or 0 percent of the cell population express the cell marker MyoD.

The term "differentiation media" as used herein preferably refers to cell culture media which induce fusion in multinucleated fusion competent cells or myogenic progenitor cells as e.g. myoblasts. However, said term may also refer to cell culture medium not comprising any substances necessary for the induction of fusion, in case the multinucleated fusion competent cells or myogenic cells can fuse without a respective induction.

The term "cell growth medium" as used herein preferably refers to any medium suitable for the incubation of mammalian cells such as myogenic progenitor cells, which allows the attachment of said mammalian cells on the surface of an incubation container (flask or dish).

The term "incontinence episode" or "IE" as used herein preferably refers to an event of uncontrolled, unexpected and/or involuntary loss of liquid or solid feces through the rectum. Incontinence episodes can be classified as "traces", "little" or "more". This classification may be made by having a patient to fill a diary which allows to track time and date of when incontinence events happen and preferably allows to classify the size and/or volume of the episode. Said diary is preferably kept for at least 1 week, more preferably for about 1 to about 4 weeks, for about 1 to about 3 weeks or for about 1 to about 2 weeks.

The term "weekly incontinence episode frequency" also termed "weekly IE frequency" also termed "weekly IEF" as used herein preferably refers to the number of incontinence episodes that occurred during a 28-days period which has been normalized to a period of 7 days calculated as follows:

$$weekly\ IEF = \frac{Number\ of\ incontinence\ episodes\ reported\ during\ the\ period}{number\ of\ days\ completed\ during\ the\ period}\ x\ 7$$

The incontinence episodes herein classified as "traces" preferably corresponds to episodes of anal incontinence whereby liquid or solid feces unexpectedly leaked from the rectum of a subject, whereby the amount of feces is so low that it appears as staining or marks in the linen of the subject. Such "traces" may not require the patient to change the underwear as the amount is soaked up by the linen of the underwear.

The term "responder", as used herein, preferably refers to a patient having at least 50% reduction of its weekly IEF calculated by comparing two incontinence diaries, of which one was filled during a specific time frame before a specific intervention (e.g. methods for use of myogenic progenitor cells according to the present invention) and one was filed at a specific timeframe after a specific intervention. Such time frame before and/or after intervention preferably is 1 week, more preferably 2 weeks, even more preferably 3 and even more preferably 4 weeks long.

The incontinence episodes herein classified as "little" preferably refers to episodes of anal incontinence whereby liquid or solid feces unexpectedly leaked from the rectum of a subject, whereby the amount of feces is more than appearing as staining or marks in the linen of the subject, but its volume is only little in comparison to what a normal defecation would result in. Such episodes may require the patient to change the underwear as the amount cannot be soaked up by the linen efficiently and otherwise would lead to discomfort.

The incontinence episodes herein classified as "more" preferably refers to episodes of anal incontinence whereby liquid or solid feces unexpectedly leaked from the rectum of a subject, whereby the amount of feces may be more than appearing as staining or marks in the linen of the subject, and its volume may be comparable to what a normal defecation would result in. Such episodes preferably require the patient to change both the underwear and secondary clothing as both will be affected and otherwise lead to severe discomfort.

The term "incremental squeeze pressure" as used herein preferably corresponds to the result of subtracting the anal resting pressure from the maximal anal squeeze pressure.

The term "conditions associated with incontinence" as used herein preferably corresponds to the results of a medical practitioner obtained when examining a subject at risk for developing anal incontinence or subject having developed anal incontinence. Such subjects might be examined for their anal sphincter apparatus e.g. by ultrasound and/or manometry in order to define the status of muscular damage and/or muscular atrophy. Further the examiner might address whether conditions such as Pelvic floor dysfunction, Nerve damage or Loss of storage capacity are associated with a risk to develop anal incontinence or are associated with the developed anal incontinence. Also classified as conditions associated with incontinence, might be diarrhea and constipation.

The term "pelvic floor dysfunction" as used herein preferably corresponds to the condition where a subject is unable to correctly relax and coordinate the muscles of the pelvic floor to urinate or to have a bowel movement. Pelvic floor organs affected by pelvic floor dysfunction might be the bladder, the uterus and vagina, the prostate and the rectum. Painful urination, incontinence or consistent urgency to go to the toilet may be signs of pelvic floor dysfunction.

The term "muscle damage" as used herein preferably corresponds to a deficiency of the muscle of the anal sphincter apparatus. Such muscle damage may be detected by a physician when conducting ultrasound or any other useful examination technique to visualize the anal sphincter muscles. Such muscle damage may affect for example the external and/or internal anal sphincter muscle. Typical examples of muscle damage visible by ultrasound are scar formations following sphincter rupture during childbirth.

The term "muscle atrophy" or alternatively "atrophy" as used herein preferably corresponds to a loss in muscle mass of the anal sphincter muscles. Such loss in muscle mass may go along with a deposition of connective and/or fat tissue between the muscle cells. Muscle atrophy might occur during ageing or when muscle remain unused. A physician might be able to detect muscle atrophy of the anal sphincter apparatus e.g. by ultrasound.

The term "nerve damage" as used herein preferably corresponds to the lack of functionality of nerve tissue that would normally functionally innervate the anal sphincter musculature and is part of the sphincter apparatus. Such nerve could be the nervus pudendus and/or the nerves leading to the nervus pudendus. The term may further correspond to deficiency of any nerve tissue that might be associated with incontinence.

The term "loss of storage capacity" as used herein preferably corresponds to an abnormality in the tolerable volume of the rectum. Loss of storage capacity might occur if the rectum is stiff and lost normal elasticity due to surgery, inflammation, or radiation therapy. Loss of storage capacity of the rectum may lead to deficiency in the ability to of the rectum to stretch (i.e. elongate) and thus results in leakage of excess stool through the rectum.

The term "duration of incontinence" as used herein preferably corresponds to the time that passed since the date of onset- or diagnosis of incontinence. The date of onset of incontinence preferably corresponds with the date of onset of symptoms, i.e. the first occurrences of incontinence episodes.

The date of diagnosis of incontinence preferably corresponds to the date when a medical practitioner diagnoses a patient with incontinence. Said date of diagnosis of incontinence preferably is identical with the date of onset of symptoms or alternatively is later than the date of onset of symptoms. In case of fecal incontinence, the term "duration of incontinence" might also be termed "duration of FI" or "incontinence duration". If the duration of incontinence is measured from the diagnosis of incontinence, the term "duration of incontinence" may also refer to "time since first diagnosis of FI".

The term "severity of incontinence" as used herein preferably corresponds to the amount of burden on a subject having developed anal incontinence. Such severity may be expressed by the number of weekly incontinence episodes, by the impact of the incontinence on the patient's life, the fecal incontinence quality of life score and/or the Wexner score. Preferably, the Wexner score corresponds to the Continence Grading Scale as disclosed in Table 3 of Jorge et al., 1993.

The term "stimulating" as used herein preferably corresponds to any action performed on the anal sphincter apparatus leading to muscular contractions. Stimulating as used herein could be performed comprising voluntary contraction of the anal sphincter muscles or by electrical stimulation. The latter might be performed by stimulating the nerves innervating the anal sphincter muscles or by directly stimulating the anal sphincter muscles, such as the external and/or internal anal sphincter muscle.

The term "comprising" as used herein shall not be construed as being limited to the meaning "consisting of" (i.e. excluding the presence of additional other matter). Rather, "comprising" implies that optionally additional matter may be present. The term "comprising" encompasses as particularly envisioned embodiments falling within its scope "consisting of" (i.e. excluding the presence of additional other matter) and "comprising but not consisting of" (i.e. requiring the presence of additional other matter), with the former being more preferred.

The term "AChE positive" or "AChE+" as used herein preferably refers to a cell or cell population showing an (i.e. a positive) acetylcholinesterase enzymatic (AChE) activity. In particular, such an AChE activity is positive if $2*10^5$ cells show an AChE activity of about $20\,mU_{rel}$ to about $1000\,mU_{rel}$, more preferably about $30\,mU_{rel}$ to about $800\,mU_{rel}$, and even more preferable about $50\,mU_{rel}$ to about $700\,mU_{rel}$. Preferably, said $2*10^5$ cells have been cultivated in skeletal muscle differentiation medium as e.g. described in the examples herein. The unit "$mU_{rel}$" refers to a relative "mU/ml" of $2*10^5$ cells measured 60 minutes after addition of Acetylthiocholine iodide (ATI) and 5,5'-Dithiobis(2-nitrobenzoic acid) (DTNB), in respect of a straight-line equation obtained by a dilution series of an AChE stock solution ranging from 4-500 mU/ml under the same conditions except for that the OD of the dilutions of the stock solution is already measured 6-8 min, preferably 6, 7, or 8 min, after addition of ATI and DTNB. The term "$mU_{rel}$" may also refer to a relative "mU/ml" of $2*10^5$ cells as measured and calculated in Example 3.

The term "AChE negative" or "AChE-" as used herein preferably refers to a cell or cell population having no (i.e. a negative) AChE activity. In particular, such an AChE activity is negative if $2*10^5$ cells show an AChE activity of about $0\,mU_{rel}$ to about $19\,mU_{rel}$, wherein the cells have preferably been cultivated in skeletal muscle differentiation medium as e.g. described in Example 2 herein.

The term "multipotent" as used herein preferably refers to the differentiation potential of mesenchymal cells characterized by in vitro differentiation potential at least towards adipogenic, chondrogenic- and osteogenic- lineages.

The term "oligopotent" as used herein preferably refers to the differentiation potential of cells characterized by in vitro differentiation potential limited to the myogenic lineages such as smooth-, striated-, and/or cardiac muscle.

The term "myogenic differentiation potential" as used herein preferably refers to the ability of a cell to express detectable amounts of proteins known to be expressed by myogenic cells *in vivo,* such as but not limited to one or more of the following markers of Desmin and Myosin. Tests for expression of such markers in *in vitro* cultivated cells are well known to the skilled person in the art. Such tests preferably involve flow cytometric and/or other immunocytochemical assays including fluorescent immunostainings and western blotting. Alternatively, preferred is the use of the term myogenic differentiation potential referring to the ability of a cell (e.g. myogenic progenitor cell) to form multinucleated myotubes. Such myotubes are known to the skilled person in the art as muscle cells comprising preferably at least 3 separate nuclei. Formation of such myotubes by originally single nucleated cells in e.g. a differentiation medium may thus be used as a test for myogenic differentiation potential.

The term "myotube" as used herein, preferably refers to a muscle cell comprising at least 3 separate nuclei. Preferably, such myotube is formed by fusion of single nucleated cell (e.g. myogenic progenitor cells).

The term "neurogenic differentiation potential" or "neuronal differentiation potential" as used herein preferably refers to a cell population expressing neuronal markers such as but not limited to one or more of the markers A2B5, TUJ1, NCAM, Nestin or equivalent markers. Preferably said term refers to a cell population of which at least 60% of the cells express A2B5, more preferably at least 60% of cell express A2B5 and TUJ1 or at least 60% of cells express A2B5 and NCAM or at least 60% of cells express A2B5 and Nestin or at least 60% of cells express TUJ1 and NCAM or at least 60% of cells express TUJ1 and Nestin or at least 60% of cells express NCAM and Nestin. More preferably said term refers to cell populations of which at least 60% of cells express A2B5, TUJ1 and NCAM or at least 60% of cell express TUJ1, NCAM and Nestin or at least 60% of cells express A2B5, NCAM and Nestin. Even more preferably said term refers to a cell population of which at least 60% of cells express A2B5, TUJ1, NCAM and Nestin. Tests for expression of such markers in *in vitro* cultivated cells are well known to the skilled person in the art. Such tests preferably involve flow cytometric and/or other immunocytochemical assays including fluorescent immunostainings and western blotting.

The term "adjacent" as used herein preferably refers to a distance between a pre-specified and an actual position reached e.g. when injecting a pharmaceutical ingredient into a patient. Preferably the "adjacent" position is directly neighboring the pre-specified position, e.g. one type of tissue directly neighboring another type of tissue, i.e. by directly contacting each other Preferably the term "adjacent" refers to any type of tissue that is indirectly or more preferably directly attached to the muscles of the anal sphincter apparatus, such as but not limited to the mucosa, submucosa, mucosa muscularis or anal canal epithelium. Alternatively preferred, the term "adjacent" refers to a distance, preferably 0.3 to 15 mm, between the injected site within a subject towards a desired site within a subject, wherein a cell, preferably myogenic progenitor cells, may migrate from the injection site to the adjacent desired site. Alternatively preferred is that "adjacent" refers to the relative distance between an actual injection site and a desired position whereas the two positions are not more distant from each other as of 5 cm, more preferably 1 cm and even more preferably less than 0.5 cm.

## DESCRIPTION OF THE EMBODIMENTS

The present invention is directed to myogenic progenitor cells (MPCs), namely myoblasts, for use in a method for the treatment of anal incontinence in a subject, wherein said subject suffers from anal incontinence for 6 months to 10 years as defined in the claims.

Subject selection

The inventors found that selecting the broad patient population (ITT) of Example 5 and treatment of patients within this patient population according to the methods of the present invention with MPCs leads to significantly higher reduction in incontinence symptoms than placebo treatment of a comparable patient population within the ITT population (Figure 1). However, statistically significancy only demonstrates that the effect is not random (e.g. not caused by placebo effects or effects unrelated to the MPCs). Secondly the observed effects need to be clinically relevant. The higher the reduction in weekly IEF and/or the higher the rate of responders, the more clinically relevant is the outcome of the treatment method in a specific patient population. The applicant found that by selecting different subpopulations of patients from ITT as described in Example 7, treatment effects were altered.

Selecting a subgroup of patients according to Example 7 from ITT population of Example 5 which were defined suffering from IE for 10 or less than 10 years (TPP1 population), led to the observation that in these patients, LCC and/or HCC treatment was leading to higher reduction in weekly IEF, and leading to higher responder rates than LCC or HCC treatment is doing in ITT patients (Figure 2). The inventors found that by introducing such narrow subject population, the methods for use of MPCs are more effective compared to broad patient populations in terms of effect sizes and odds ratios (Figure 8) and thus favorable compared to already known method of use of MPCs.

Thus, according to the present invention said subject suffering from anal incontinence, more preferably fecal incontinence, is suffering from said incontinence for a limited amount of time, namely for 6 months to about 10 years, more preferably limited to about 6 months to about 8 years, more preferably limited to about 6 months to about 6 years, more preferably limited to about 6 months to about 4 years and even more preferably limited to about 6 months to about 2 years. The starting point of the preferred time is either the onset of incontinence or the diagnosis of incontinence.

The present invention provides the MPCs for use in a method for treatment, wherein the subject to be treated is suffering from anal incontinence, in particular from fecal incontinence, more preferably from urge fecal incontinence and/or passive fecal incontinence, more preferably from urge fecal incontinence.

In one preferred embodiment of the present invention, the subject's anal incontinence, preferably fecal incontinence, is caused by muscle damage as e.g. described above. Preferably, said muscle damage is present as a scar within the external and/or internal anal sphincter to be visualized by endoanal ultrasound. Said muscle damage can occur in the external anal sphincter, the internal anal sphincter or both. It may be caused by (1) obstetric or accidental trauma, (2) iatrogenic trauma such as surgery or radiation, (3) neurogenic diseases such as multiple sclerosis or diabetes mellitus or (4) muscle atrophy associated with aging. Obstetric trauma is the main cause of incontinence in women since up to 9 % of vaginal deliveries lead to rupture of the anal sphincter.

Said subject is suffering from fecal incontinence by well-defined severity, namely by more than 2, more preferably more than 3, even more preferably more than 4, even more preferably more than 5, even more preferably more than 6, even more preferably more than 7, even more preferably more than 8, even more preferably more than 9, even more preferably more than 10, and even more preferably more than 11 weekly incontinence episodes, wherein all episodes classified as "little" and/or "more" are taken into account.

The inventors found that patients with increasing severity (i.e. more baseline IEF) are more responsive to MPCs treatment (Figure 7). The inventors found that selecting such narrow subgroup of patients according to Example 7 which in addition to the definitions of TPP1 is defined by having more than 6 weekly IEF before treatment (TPP2) leads to an increased reduction of weekly IEF and increased responder rate as well as effect size and odds ratios in said patient population following administration of MPCs (LCC or HCC) compared to what was observed in the broader population of patients (ITT, TPP1) (Figure 2). This is additionally underlined by an overall increased effect size and odds ratio (Figure 8).

Further, the inventors found that treatment of anal or fecal incontinence according to the present invention is most effective on episode types defined as "more" and "little" compared to "traces" (Table 3). Thus, said severity is defined by suffering from a specific amount of weekly incontinence episode frequency. According to the invention, episode frequency for assessing severity is limited to incontinence episodes defined as "little" and "more", thereby excluding "traces". The inventors found that a subpopulation of TPP1 defined by incontinence severity before treatment to as more than 2 weekly incontinence episodes of "little" or "more" (TPP3) is more responsive to treatment by LCC or HCC than other patient populations (ITT, TPP1 and TPP2) as exemplified in Example 7 by increased effect sizes and odds ratios (Figure 8).

Thus, according to the present invention, MPCs are for use in a method for treatment of anal incontinence in a subject, wherein said subject has a severity of incontinence defined as more than 2, more preferably more than 3 and even more preferably more than 4 weekly incontinence episodes classified as "little" or "more" prior to treatment.

Prior to treatment preferably refers to a treatment according to the present invention. Thus, the weekly incontinence episodes are preferably classified prior to any administration of MPCs as described herein. Preferably, "prior to treatment" does not include any treatment attempts other than the treatment according to the present invention as e.g. conservative treatment by Loperamide or surgical treatment by graciloplasty or sphincteroplasty.

The inventors found that selecting a subgroup of patients according to Example 7 which in addition to the definitions of TPP1 is defined by having more than 6 weekly IEF before treatment (TPP2) leads to an increased reduction of weekly IEF and increased responder rate in said patient population following administration of MPCs (LCC or HCC) compared to what was observed in the broader population of patients (ITT, TPP1) (Figure 2). This is additionally underlined by an overall increased effect size and odds ratio (Figure 8). Thus, this narrow selection of patient population is favorable compared to the broad selection of patients already known in the art.

Thus, in a highly preferred embodiment of the present invention, the MPCs are for use in a method of treatment of anal incontinence, more preferably fecal incontinence, in a subject, wherein said subject

(i) suffers from anal incontinence, more preferably fecal incontinence, for 6 months to 10 years, and
(ii) has more than 6 weekly incontinence episodes prior to treatment, and
(iii) has a severity of incontinence defined as more than 2, more than 3 or more than 4 weekly incontinence episodes classified as little or more prior to treatment.

The inventors found that combination of limited duration of incontinence and increased severity of FI in terms of weekly incontinence episode frequency, wherein episodes classified as "traces" are excluded, is a suitable selection process of Example 7 to select patients which benefit much better from the method of treating fecal incontinence using MPCs in accordance with the present invention. The inventors found that selecting such narrow subgroup of patients according to Example 7 which in addition to the definitions of TPP1 is defined by having more than 2 weekly IEF, wherein traces do not account as incontinence episodes, before treatment (TPP3) leads to an increased reduction of weekly IEF and increased responder rate in said patient population following administration of MPCs (LCC and/or HCC) compared to what was observed in the broader population of patients (ITT, TPP1) (Figure 2). This is additionally underlined by an overall increased effect size and odds ratio (Figure 8). Thus, this narrow selection of patient population is favorable compared to the broad selection of patients already known in the art.

The inventors found that selection of a subgroup of fecal incontinent patients which suffer from fecal incontinence associated with muscle damage in combination with 10 or less than 10 years since first diagnosis of FI as well as more than 2 weekly IEF prior to treatment, are especially responsive to treatment of their symptoms by the methods of the present invention (Example 7, Figure 9 and Figure 10).

In a preferred embodiment, the subject is 18 years of age or older. In a further preferred embodiment, the subject is suffering from fecal incontinence for more than 6 months, which is confirmed at screening by relevant medical history and anorectal examination. In a further preferred embodiment, the subject has a Wexner score >9 and with at least 3 episodes of fecal incontinence per week as measured in a bowel diary over a certain period of time as e.g. 2, 3 or 4 weeks. In a further preferred embodiment, the subject has with a minimum of 3 non-gaseous incontinence episodes per week measured using a diary as mentioned above.

In a preferred embodiment, the subject is has not undergone any anorectal surgery within the last 6 months prior to the date of administering the MPC. In a further preferred embodiment, the subject has no more than one previous overlap repair surgery. The term "overlap repair" as used herein refers preferably to a primary overlap repair as usually performed to correct an acute damage in the event of a tear at birth in the art. Preferably, said term also refers to a comparable procedure as performed in a primary overlap repair but at a later time (without acute damage) which procedure may also be called "sphincteroplasty". In a further preferred embodiment, the subject has no more than 2 anorectal surgical procedures in total (e.g. primary repair after delivery and one overlap repair later-on or in- and explantation of a permanent neurostimulation system). In a further preferred embodiment, the subject has no overlap repair and associated early atrophy of external anal sphincter. In a further preferred embodiment, the subject has no history of artificial anal sphincter (AAS) surgery. In a preferred embodiment, the subject has no trans- or perianal injection of any bulking products. In a preferred embodiment, the subject has not undergone radiation therapy of the bowel and pelvis. In a preferred embodiment, the subject has not undergone chemotherapy within last 5 years prior to the date of administering the MPC. In a preferred embodiment, the subject has no chemotherapy related neuropathy of the bowel and pelvis. In a preferred embodiment, the subject is not under immunosuppressive therapy. In a preferred embodiment, the subject has no diagnosis of chronic inflammatory bowel disease. In a preferred embodiment, the subject has no diagnosis of current and anal fistula disease. In a preferred embodiment, the subject has no chronic diarrhea. In a preferred embodiment, the subject has no acute anal sphincter injury including obstetric and other trauma, acute disc prolapses, or neurological diseases (spinal cord injury, multiple sclerosis, Parkinson's disease, stroke, etc.). In a preferred embodiment, the subject has no uncontrolled diabetes mellitus type I or II, or suffering from diabetic peripheral neuropathic pain. In a preferred embodiment, the subject is not diagnosed with human immunodeficiency virus (HIV), acute or chronic viral hepatitis HCV, acute or chronic viral hepatitis HBV, active Syphilis, HTLV (e.g. tested upon risk assessment by investigator). In a preferred embodiment, the subject has no implantations of metal components in the electrical stimulation treatment area. In a preferred embodiment, the subject has no chronic constipation and/or overflow incontinence.

In a preferred embodiment, the subject has a condition associated with incontinence of muscle damage, pelvic floor dysfunction, nerve damage, loss of storage capacity, or atrophy. In a preferred embodiment, the subject's fecal incontinence is a passive incontinence, an urge incontinence or a fecal seepage. In a highly preferred embodiment, the subject has a condition associated with fecal incontinence of muscle damage, atrophy and/or urge incontinence.

The MPCs for use according to the present invention are administered into or adjacent to the anal sphincter apparatus of said subject, preferably into the external anal sphincter muscle, into the internal anal sphincter muscle and/or into the puborectalis muscle, preferably as further described herein. The administration is performed by injection. The process of injection process may be, but is not limited to, a static injection, i.e., the injection device remains at the position reached. Alternatively, the injection process may be dynamic.

In any of the above preferred embodiments of the present embodiments, said method further comprises stimulation of the anal sphincter apparatus prior to and/or following the administration of MPCs. Preferably, said stimulation comprises at least 2 weeks, more preferably at least 4 weeks, of anal sphincter apparatus stimulation after administration of MPCs, more preferably at least 2 weeks, more preferably at least 4 weeks, of anal sphincter apparatus stimulation prior to and at least 2 weeks, more preferably at least 4 weeks, of anal sphincter apparatus stimulation after administration of MPCs.

Preferably, said stimulation is performed by Kegel exercise and/or by electrical stimulation, more preferably by Kegel exercise and/or percutaneous electrical stimulation. Said percutaneous electrical simulation is preferably performed daily, more preferably twice, more preferably trice per day. Preferably any of said stimulation is performed for at least 10 minutes, more preferably for at least 20 minutes. Also preferred is that stimulation is conducted multiple times per day, more preferably 1-10, more preferably 1-5 and even more preferably 3 times per day.

The electrical stimulation is preferably performed for a specific amount of time per day, for example at least 5, preferably at least 15 and more preferably at least 20 minutes per session. Preferably a patient conducts stimulation 3 times per day each for about 1-60 minutes, preferably about 10-30 minutes, more preferably about 20 minutes. Preferably electrical stimulation comprises use of a recto anal probe attached to a stimulator. Preferably the said recto anal probe is inserted into the rectum of a subject during stimulation. Preferably said stimulator is programmed to transmit electrical pulses to the probe, wherein the pulses preferably are biphasic or monophasic. Preferably, said pulses are embedded in a sequence of pause, increase, plateau, decrease, pause. Preferably said biphasic or monophasic pulses have at said plateau a frequency of about 10 to about 100 Hz, more preferably about 25 Hz to about 75 Hz, more preferably about 50 Hz. Preferably each plateau phase has the same duration as each increase or decrease phase. Preferably each pause, increase, decrease, or plateau phase lasts for about 1 sec to about 15 sec, more preferably about 2 sec to about 8 sec, more preferably about 4 sec. Preferably, strength of electric current at plateau phases is set to about 1 mA to about 1000 mA, more preferably to about 10 mA to about 500 mA, more preferably about 50 mA to about 250 mA, more preferably to about 75 mA to about 150 mA, more preferably to about 100 mA. Preferably, biphasic or monophasic pulses have a width of about 50 $\mu$s to about 500 $\mu$s, more preferably a width of about 150 $\mu$s to about 300 $\mu$s, more preferably about 200 $\mu$s to about 300 $\mu$s, more preferably 250 $\mu$s.

Kegel exercise is preferably conducted by a subject by voluntary contraction of the pelvic floor muscles. Preferably, a contraction is hold for about 1 sec to about 10 sec, more preferably for about 2 sec to about 5 sec, more preferably for about 3 sec. After a contraction, preferably the muscles are relaxed for about 1 sec to about 10 sec, more preferably for about 2 sec to about 5 sec, more preferably for about 3 sec. Preferably a sequence of said contraction and said relaxation is repeated for 1 to 20, more preferably 5 to 15, more preferably about 10 times. Preferably each set of said number of sequences is preferably repeated for 1 to 10, more preferably 3 to 5, more preferably 3 times every day.

The inventors found that combination of electrical stimulation and cell injection (e.g. HCC or LCC treatment according to Example 1) is more favorable than electrical stimulation and sham injection alone (e.g. PBO treatment according to Example 1) (Figure 1), suggesting that combination of MPCs and electrical stimulation is an effective treatment for incontinence.

Selection of Cell Dose

Preferably, the MPCs are for use in a method for the treatment of anal incontinence in a subject, wherein the method comprises the administration of an effective amount of MPCs, preferably an administration of about 1 to about 200 million MPCs, preferably of about 4 to about 60 million MPCs, more preferably of about 4 to about 6 million MPCs or about 40 to about 60 million MPCs.

The present inventors found out that following injection of 40-60 million MPCs of (HCC) a significantly higher reduction of the weekly incontinence episode frequency compared to placebo injection (PBO) occurs at 6 months post treatment, when counting tracing as incontinence episodes (Figure 1A), and at 12 months post treatment, when excluding traces as incontinence episodes (Figure 1B) in a broad patient population (ITT). A trend for higher reduction in weekly incontinence frequency by HCC than PBO was observed over the whole study period of Example 5 independent of inclusion/exclusion of traces as incontinence episodes (Figure 1). Furthermore, a significantly higher responder rate in HCC treated compared to PBO treated ITT at 1 and 12 months, when excluding traces (Figure 1D) and a consistently higher number of responders in LCC and HCC group compared to PBO group was visible when including traces in the ITT patient population (Figure 1C). Also preferably is an amount of 4-6 million cells per patient according to the present embodiment. The applicant found that a trend for higher reduction of both weekly IEF and increased responder rate following treatment of ITT patients with 4-6 million cells (LCC), compared to PBO treatment was observed (Figure 1). This trend became significant when using 4-6 million cells for fecal incontinence patients suffering from no longer than 10 years from fecal incontinence. The inventors found that for such patients, LCC implantation was significantly reducing IEF from baseline to 12 months post treatment (Figure 2A and B) as well as clinically relevant by reducing weekly IEF (excluding traces) in most patients by at least 50% (Figure 2D). The inventors further found that patients injected with 40-60 million cells were found to have a tendency for higher reduction in fecal incontinence symptoms (weekly IEF) compared to patients injected with 4-6 million cells.

Injection Procedure

According to the present invention, the MPCs are injected into or adjacent to the anal sphincter apparatus of a subject. Preferably, said cells are injected into or adjacent to the external anal sphincter muscle, into or adjacent to the internal anal sphincter muscle and/or into or adjacent to the puborectalis muscle. More preferably, said cells are injected into the external anal sphincter muscle, internal anal sphincter muscle or puborectalis muscle. Most preferably, said cells are injected into the external anal sphincter muscle. The present inventors found that injection of MPCs into the external anal sphincter is effective compared to placebo treatment (Figure 1). The inventors found that injecting cells, isolated according to Example 1, into subjects in need according to Example 5 is successful. The injection site was chosen as the external anal sphincter, which belongs to the anal sphincter apparatus.

In one preferred embodiment of the present invention, MPCs are injected into a given tissue or site of injury, whereby MPCs comprise a therapeutically effective number of cells in solution or suspension, e.g., about $1 \times 10^6$ to about $2 \times 10^8$ cells. Said number of cells for injection preferably is suspended in a solution of about 0.1 mL to about 100 mL, more preferably about 1 mL to about 10 mL, more preferably about 3 mL to about 6 mL and even more preferably in 6 mL. The injection solution is a physiologically acceptable medium, with or without serum. Physiological acceptable medium can be by way of nonlimiting example physiological saline, or a phosphate buffered solution.

In one preferred embodiment of the present invention, MPCs injection into the anal sphincter apparatus is performed as a treatment for anal incontinence to enhance, improve, and/or repair the external and/or internal anal sphincter. Preferably, the MPCs are injected into or adjacent to the external and/or internal anal sphincter and survive and differentiate into mature muscle cells to augment the sphincter and/or improve sphincter function. The feasibility and long-term survival of MPCs in accordance with this embodiment has been shown before (Messner et al., 2021; Thurner et al., 2020).

In one preferred embodiment of the present invention the effective cell number is administered in aliquots of one or multiple cell suspension(s). Preferably multiple aliquots are injected per patient, more preferably 2 to 50 aliquots, more preferably 2-20 and even more preferably 5-15 aliquots Preferably a total of 0.1-100 ml, more preferably 1-10 ml, even more preferably 3-6 ml is injected per patient. The inventors found that such number of aliquots and volumes are effective (i.e. superior to placebo application) and clinically relevant (i.e. most patients having at least 50% reduction in weekly IEF) (Figure 1 and Figure 2).

According to the present invention, the MPCs are administered by one or more injections into and/or adjacent to the anal sphincter apparatus of said subject, preferably by one or more injections into the external anal sphincter muscle, the internal anal sphincter muscle and/or into the puborectalis muscle. Preferably, MPCs for use according to the present invention are injected into the internal anal sphincter muscle of a subject, wherein the subject further is characterized by suffering from passive- or passive- and urge fecal incontinence. Preferably, MPCs for use according to the present invention are injected into the external anal sphincter muscle and/or puborectalis muscle, more preferably the external anal sphincter muscle, of a subject, wherein said subject further is characterized by suffering from urge- or urge- and passive fecal incontinence. Preferably, MPCs injection is performed at multiple locations throughout the anal sphincter apparatus.

Preferably, MPCs are injected at one or multiple sites, more preferably at about 2 to about 20, more preferably at about 6 to about 18, more preferably at about 10 to about 15 sites, even more preferably at about 12 sites. The inventors found that distributing the injection of MPCs of Example 1 in patients according to Example 5 is effective and significantly more effective than placebo treatment (Figure 1).

<u>MPCs for use in the present invention</u>

The present invention provides MPCs, namely myoblasts, for use according to the present invention. In one embodiment of the present invention MPCs are characterized as "muscle-derived cells" already disclosed in EP2120976B1, which are myoblasts which reside in muscle tissue. In particular, the cells for use in the present invention are capable to fuse (form a syncytium of at least three cells) and to establish an oriented, contractile cytoskeleton (actin-myosin sequence) *in vitro* and/or *in vivo.* In accordance with the present invention, MPCs including myoblasts, may be primary cells or cultured cells. They may be histocompatible (autologous) or non-histocompatible (allogeneic) to the recipient, including humans. Particular embodiments of the present invention are autologous myoblasts which will not be recognized as foreign to the recipient. In this regard, the myoblasts can be matched vis-á-vis the major histocompatibility locus (MHC or HLA in humans). Such MHC or HLA matched cells may be autologous. Alternatively, the cells may be from a person having the same or a similar MHC or HLA antigen profile. The patient may also be tolerized to the allogeneic MHC antigens or the cells might be engineered to lack MHC proteins, thereby becoming immune tolerable to an originally HLA mismatched recipient.

In another embodiment of the present invention, the MPCs are lacking MHC Class I and/or II antigens, such as described in U.S. Patent 5,538,722.

In a highly preferred embodiment, MPCs are a cell population of skeletal muscle derived cells (SMDCs), wherein at least 60 % of the cells are positive for CD56, at least 80% of the cells are positive for CD90, and at most 10% of the cells are positive for CD34. Preferably, also at least 60% of the cells are positive for A2B5 and CD105. Preferably said cell population of SMDC is negative for Sca-1, in particular wherein at most 10 % or 0% of the cells express Sca-1. Preferably, also at least 60% of the cells are positive for desmin. Preferably said cell population of SMDC is negative for MyoD, in particular wherein at most 5% or 10 % of the cells express for MyoD.

In a further preferred embodiment, MPCs are a cell population of SMDCs, wherein about 64 % to about 99.9 % of the cells are positive for CD56, about 80 % to about 99.9 % of the cells are positive for CD90, and about 0 % to about 9 % of the cells positive for CD34. Preferably, also at least 60% of the cells are positive for A2B5 and CD105. Preferably said cell population of SMDC is negative for Sca-1, in particular wherein at most 10 % or 0% of the cells express Sca-1. Preferably, also at least 60% of the cells are positive for desmin. Preferably said cell population of SMDC is negative for MyoD, in particular wherein at most 5 % or 10 % of the cells express for MyoD.

In another embodiment of the present invention, MPCs are characterized as the "skeletal muscle derived cells" (SMDC) disclosed in WO 2019/115790 (Thurner et al., 2019, p. 115790). SMDC, exhibit preferably a characteristic expression pattern. Preferably, more than about 60%, 70%, 80%, 90 %, 95 % or 98% of said SMDC express CD56 and A2B5. Preferably, said SMDC do not express CD34, Sca-1 and MyoD. Thereby, the term "do not express" means that preferably less than 40%, 30%, 20%, 10%, 5% or 2% of the SMDC express said markers. In a preferred embodiment 0% of said SMDC express Sca-1.

In a further preferred embodiment, the MPCs are a cell population of SMDCs, wherein the population comprises at least 90% CD56 positive, at least 90% A2B2 positive, at least 90% CD105 positive and at least 90% desmin positive cells and less than 10% of the population express CD34, Sca-1 and MyoD. In a preferred embodiment 0% of said SMDC express Sca-1.

The expression pattern of SMDC as described above can be used to determine the myogenicity index of the cell culture without the requirement of differentiation. Thus, said expression pattern of SMDC can be used to verify, whether skeletal muscle derived cells can be used for the treatment of a muscle dysfunction, in particular for the treatment of incontinence such as urinary and/or anal incontinence.

In another embodiment of the present invention the MPCs as described herein are characterized as "myogenic progenitor cells" disclosed in WO 2020/193460. Preferably these cells are characterized by the positive expression of CD56 and CD90, and the negative expression of CD34. In a further preferred embodiment of the invention, the MPCs are oligopotent. The inventors found that such cells are useful for the methods of the present invention (Figure 1).

In a highly preferred embodiment, the MPCs for use in the method according to the present invention are characterized by a positive expression of the markers of CD56 and CD90, in particular wherein at least 60 %, more preferably at least 80%, more preferably at least 95% of the MPCs express CD56 and at least 60%, more preferably at least 80%, more preferably at least 95% of the myogenic progenitor cell express CD90. Preferably, about 60 to about 99.9 % of said MPCs express CD56 and about 80 to about 99.9 % of said MPCs express CD90. Preferably, said MPCs are further characterized by a negative expression of the marker CD34 and/or Sca-1, in particular wherein at maximum 10 %, more preferably at maximum 5%, more preferably at maximum 1% of the MPCs express CD34 and/or Sca-1. Even more preferably, MPCs are further characterized by a positive expression of the marker Desmin, in particular wherein at least 60%, more preferably at least 80%, more preferably at least 95% of cells are Desmin positive.

In a preferred embodiment, the MPCs for use in a method according to the present invention are composed of a cell population having the following marker expression characteristics:

About 60 to about 99.9% of said cell population (i.e. of the MPCs) express the cell marker CD56, about 60 to about 99.9% of said cell population express the marker CD90 and about 0 to about 15% of said cell population express the marker CD34. More preferably, about 60 to about 99.9% of said cell population express the cell marker CD56, about 70 to about 99.9% of said cell population express the cell marker CD90 and about 0 to about 10% of said cell population express the cell marker CD34. More preferably, about 80 to about 99% of said cell population express the cell marker CD56, about 80 to about 99.9% of said cell population express the marker CD90 and about 0 to about 5% of said cell population express the marker CD34.

The MPCs for use according to the present invention may further have the following marker expression characteristics:

About 60 to about 100% of said cell population express the cell marker Desmin, and/or about 60 to about 100 % of said cell population express the marker CD105. More preferably, about 60 to about 99.9% of said cell population express the cell marker desmin, and about 90 to about 100% of cells express the cell marker CD105. Preferably, about 60 to about 100 % of said cell population further express the cell marker A2B5.

In a further particularly preferred embodiment, the MPCs have the following marker expression characteristic:

About 60 to about 100% of said cell population express the cell marker CD56, about 60 to about 100% of said cell population express the cell marker CD90, about 0% to about 10% of said cell population express the cell marker CD34, about 60 to about 100 % of said cell population express the cell marker desmin, about 60% to about 100% of said cell population express the cell marker A2B5 and about 60 to about 100% of said cell population express the cell marker CD105. Preferably, about 0% to about 10% of said cell population show expression of the cell marker Sca-1.

In a further preferred embodiment of all embodiments for MPCs as described above, about 0 to about 10% of the MPCs express MyoD and/or about 0 to about 10% of the MPCs express Sca-1.

In one particular preferred embodiment the MPCs for use according to the present invention have the following marker expression characteristic:

About 60 to about 100% of said cell population express the cell marker CD56, about 80 to about 99.9% of said cell population express the cell marker CD90, about 0% to about 10% of said cell population express the cell marker CD34, about 60 to about 100 % of said cell population express the cell marker desmin, about 60% to about 100% of said cell population express the cell marker A2B5, about 60% to about 100% of said cell population express the cell marker CD105, and about 0 to about 10% of said cell population express the cell marker MyoD and/or about 0 to about 10% of said cell population express the cell marker Sca-1.

In another preferred embodiment of the present invention, MPCs for use in the method of the present invention are characterized by their differentiation potential toward cell lineages. Preferably, MPCs are characterized as comprising myogenic differentiation potential *in vitro* and/or *in vivo,* whereby differentiation potential is defined as the ability to augment, form or become mature muscular tissue, i.e. by formation of multinucleated myofibers or i.e. by electrophysiological coupling of multiple single nucleated cells. Preferably, MPCs can augment, form, or become mature muscle tissue *in vitro* and/or *in vivo.* Preferably MPCs can augment, form, or become mature skeletal *in vitro* and/or *in vivo.* MPCs further are characterized by their ability to self-renew by mitosis in culture and the ability to exit mitosis after administration into a subject.

In another embodiment of the present invention MPCs are characterized as having myogenic and neurogenic differentiation potential. Cells comprising myogenic and/or neurogenic potential may be isolated from muscle tissue, preferably by obtaining a muscle biopsy. Preferably cells with myogenic potential are isolated from a muscle biopsy by obtaining skeletal muscle derived cells (SMDCs).

Such cells can be tested for myogenic potential by methods known to the person skilled in the art. In order to determine the myogenic and/or neurogenic potential of MPCs according to the present invention, cells are tested positive for their AChE activity according to the examples of the present invention or any other method known by the skilled person in the art (e.g. Thurner et al. 2018).

MPCs for use according to the present invention have preferably an AChE activity of about 20 $mU_{rel}$ to about 1000 $mU_{rel}$, more preferably of about 30 $mU_{rel}$ to about 800 $mU_{rel}$, more preferably about 50 to about 700 $mU_{rel}$, wherein each AChE activity is determined per $2*10^5$ cells that have preferably been cultivated in skeletal muscle differentiation medium for preferably about 5 to about 7 days.

The cells of the selected cell dose which is to be administered into a subject as outlined above exhibit preferably a total AChE activity of about $1*10^2$ $mU_{rel\_total}$ to about $1*10^6$ $mU_{rel\_total}$, more preferably about $1*10^3$ $mU_{rel\_total}$ to about $5*10^5$ $mU_{rel\_total}$ and even more preferably about $7*10^4$ $MU_{rel\_total}$ to about $2*10^5$ $MU_{rel\_total}$. For determining said total AChE activity, AChE activity determined for $2*10^5$ cells may be extrapolated. For example, if a batch of cells have an acetylcholinesterase enzymatic activity of 50 $MU_{rel}$ per $2*10^5$ cells, it can be extrapolated, that a suspension of cells containing 50 million of such cells have a total acetylcholinesterase enzymatic activity of $1.25*10^4$ $MU_{rel\_total}$. Thus, the unit "$MU_{rel\_total}$" refers preferably to an extrapolated AChE activity of a certain number of cells, wherein the AChE activity is linear extrapolated from a relative AChE activity measured in $mU_{rel}$. In a further preferred embodiment, the total AChE activity given in $mU_{rel\_total}$ is the total AChE activity of a cell population or cell dose of MPCs relative to the AChE standard as described herein. Thus, the unit "$MU_{rel\_total}$" may also refers to a relative "mU/ml" of said cell population or cell dose of MPCs measured 60 minutes after addition of ATI and DTNB, in respect of a straight-line equation obtained by a dilution series of an AChE stock solution ranging from 4-500 mU/ml under the same conditions except for that the OD of the dilutions of the stock solution is already measured 6-8 min, preferably 6, 7, or 8 min, after addition of ATI and DTNB.

The inventors found that MPCs obtained according to the methods disclosed in the present invention harbor in case of LCC batches a mean±SD AChE activity of 241.90±151.90 $mU_{rel}$ ranging from 36 $mU_{rel}$ to 568 $mU_{rel}$, wherein said AChE activity is each determined per $2*10^5$ cells. HCC batches (n=75) were found to harbor a mean±SD AChE activity of 213±137.40 $mU_{rel}$ ranging from 49 $mU_{rel}$ to 680 $mU_{rel}$ wherein said AChE activity is each determined per $2*10^5$ cells.

Methods for obtaining myogenic progenitor cells

The MPCs, namely myoblasts, for use in a method according to the present invention are preferably isolated cells of muscle tissue. They can be obtained by methods well known to a person skilled in the art, e.g., via isolation form a muscle biopsy. Preferably cells may be isolated from muscle tissue, preferably by obtaining a muscle biopsy. Preferably a muscle biopsy is obtained from a subject by surgery. Preferably MPCs are isolated from a subject, more preferably from a human subject and even more preferably from a human subject in need, such as e.g. a subject suffering from incontinence. Preferably MPCs are isolated from a muscle biopsy of a subject or alternatively isolated from another tissue source such as but not limited to adipose tissue or connective tissue. Preferably said muscle biopsy is obtained from a skeletal muscle, more preferably from the *musculus pectoralis major, musculus biceps brachii,* or *musculus latissimus dorsi.*

MPCs are preferably obtained from a obtained muscle biopsy by a method comprising the following steps of (a) cooling of a obtained muscle biopsy; (b) processing and cooling of the sample; (c) resuspending the sample of step (b) in medium with serum comprising at least one enzyme and heating up to 38°C for 1 to 20 hours; pelleting the sample and (d) resuspending the pellet of the sample of step (c) to provide a single cell suspension from the sample of step (c), thereby obtaining MPCs. Step (a) may comprise conducting a muscle biopsy. Such muscle biopsy serving as the source of MPCs can be obtained from the muscle at the site of injury or from another area that may be more easily accessible to the clinical surgeon. In a further preferred embodiment, step (a) is conducted at a temperature lower than 16°C, preferably at a temperature range from 1 to 16°C, preferably 4 to 10°C, in particular preferred at 7°C; and for a time in the range of up to 96 hours. Thus, step (a) may be conducted at a temperature range of 1 to 16°C or at any temperature in between this range, such as at 2, 3, 4, 5, 6, 7, 8, 9, 10, 11, 12, 13, 14, 15°C, or any intermediate temperature within this range such as in the temperature range from 6 to 8°C. Alternatively, the temperature range is below 4°C, preferably in the range from 1 to 3°C. Step (a) is preferably conducted at a time in the range of up to 96 hours, or any time within this range, such as 12 to 96 hours, 12 to 72 hours, 12 to 48 hours, 24 to 96 hours, 24 to 72 hours, 24 to 48 hours, or any other intermediate range. Preferably, the processing of step (b) comprises the use of scissors, scalpel, tweezers, filter, or ball mill and a centrifuge. Said processing refers particularly to a mechanical disruption of said tissue sample. The processing of the sample is preferably performed at room temperature. The cooling in step (b) is preferably performed after the processing of the sample. The cooling in step (b) may be conducted at a temperature in the range of 1 to 16°C or at any temperature in between this range, such as at 2, 3, 4, 5, 6, 7, 8, 9, 10, 11, 12, 13, 14, 15°C, or any intermediate temperature within this range such as in the range from 4 to 8°C, or from 1 to 3°C. Step (b), in particular the cooling, may be conducted for a distinct time range, such as 2 to 48 hours, 2 to 36 hours, or 2 to 24 hours. Step (c) comprises preferably conducting the enzymatic treatment with a solution comprising any one or more selected from the group consisting of trypsin, papain, elastase, hyaluronidase, collagenase, deoxyribonuclease, and DNAse. In a preferred embodiment step (c) comprises the use of collagenase. The resuspension in step (c) comprises preferably the centrifugation of the sample of step (b), discarding the supernatant and resuspending of the cell pellet in a medium with serum comprising at least one enzyme such as trypsin. Step (c) may further comprise one or more washing steps, including vortexing of the cells in a suitable solution, such as a buffer. The sample of step (b) is preferably centrifuged after the incubation time to pellet the cells of the sample and to discard the enzyme containing supernatant. Step (c) may be conducted at a temperature in the range of 25 to 38°C, preferably 36 to 38°C. Preferably, step (d) comprises a method selected from at least one of FACS sorting, centrifugation, electrokinetic sorting, acoustophoresis sorting, bead-based cell sorting, and optical sorting. A suitable enrichment method for obtaining single cell suspension is magnetic-activated cell sorting (MACS®). The MACS method allows cells to be separated by incubating the cells with particles coated with antibodies against a particular surface antigen. Subsequently, the incubated cells are transferred on a column placed in a magnetic field. In this step, the cells which express the antigen and are therefore attached to the nanoparticles stay on the column, while other cells not expressing the antigen flow through the column. By this method, the cells can be separated positively and/or negatively with respect to the particular antigen(s). Another example for a suitable enrichment method is fluorescence-activated cell sorting (FACS®) as e.g. described in Webster et al. (Exp Cell Res. 1988 Jan; 174(1):252-65). A further optional step (e) may be conducted after step (d) including incubating of the single cell suspension obtained in step (d), wherein the incubation in step (e) is preferably conducted at a temperature in the range of 25 to 38°C, preferably 36 to 38°C, in particular preferred at 37°C, thereby obtaining adherent MPCs. This further optional incubation step allows to let the cells grow to achieve a higher amount of MPCs. After step (e) optionally a further step (f) may be conducted comprising discarding of non-adherent cells of step (e), wherein step (f) is preferably conducted after at least 6 hours to 4 days. After step (f) optionally a further step (g) may be conducted of propagating of the adherent cells of step (e), the propagating in step (h) comprises culturing of the adherent cells for 1 to 5 passages to 70 to 80% confluency.

The method of treating anal incontinence in a subject, for which myoblasts according to the present invention are for use, may comprise the steps of (a) selecting a subject at risk of developing anal incontinence, more preferably fecal incontinence, or a subject suffering from anal incontinence, more preferably fecal incontinence, according to the subject's conditions associated with incontinence (b) administering, preferably injecting, an effective amount of MPCs into or adjacent to the anal sphincter apparatus and, (c) optionally stimulating the anal sphincter apparatus prior to and/or following step (b).

Step (a) of the method may comprise selecting a subject with muscle damage as condition associated with incontinence. Alternatively or in addition, step (a) may comprise the selection of a subject with anal incontinence duration of 10 or less than 10 years or any of the further respective time ranges disclosed above. Alternatively or in addition, step (a) may comprise selecting a subject having a severity of incontinence defined as more than 6, preferably more than 7, preferably more than 8, more than 9 or more than 10 weekly incontinence episodes prior to treatment. Alternatively or in addition, step (a) may comprise selecting a subject having a severity of incontinence defined as more than 2 weekly incontinence episodes classified as little or more prior to treatment. Step (c) comprises preferably at least 2 weeks of anal sphincter apparatus stimulation after injecting MPCs or prior to and after injecting MPCs.

The MPCs, namely myoblasts, may be comprised in a pharmaceutical composition comprising a pharmaceutical acceptable excipient and/or carrier.

The pharmaceutical composition may additionally contain one or more conventional additive(s). Some examples of such additives include a physiologically acceptable buffer, albumin, collagen, laminin, and dimethyl sulfoxide.

Said pharmaceutical composition can be prepared by admixing MPCs with a pharmaceutically acceptable diluent, excipient, or carrier.

The subject to be treated according to any of the above-mentioned embodiments is preferably a human or an animal, in particular a mammal, most preferably a human.

The following examples explain the present invention but are not considered to be limiting.

Example 1- Isolation of Skeletal Muscle Derived Myogenic Progenitor Cells (MPCs)

During a clinical trial conducted by the applicant (EudraCT number: 2010-021463-32), MPCs were isolated from human fecal incontinent patients as already disclosed in WO2019115790. While for WO 2019115790, the isolation of myogenic skeletal muscle progenitor cells from samples of few patients was performed, for the clinical trial as outlined above the isolation of skeletal muscle derived myogenic progenitor cells from samples of about 170 distinct patients was performed. The quality of the samples used for said trail, therefore, may have differed partly considerable from the samples used in WO2019115790 depending on the patient and the quality of the obtained biopsy. In detail, a skeletal muscle biopsy was taken from M. pectoralis major or M. biceps brachii of each incontinent patient to be treated according to Example 5. To take the biopsy, first the skin was opened by an approximately 1 cm long incision above the muscle until the fascia of the M. pectoralis major was reached. After opening of the fascia, 1 cm$^3$ of muscle tissue (biopsy) was taken. The biopsy was directly transferred into a biopsy transportation medium precooled to approximately 4°C and comprised of Ham's F10 basal medium supplemented with Gentamicin (1-5 $\mu$g/ml final concentration). The biopsy was stored for approximately 26 hours at 1-11 °C within the biopsy transportation medium. Next, the biopsy was transferred to a petri dish filled with 1x PBS. The muscle tissue was separated from connective tissue using sterile forceps and a scalpel. Then, the muscle tissue was transferred into another petri dish filled with 1x PBS and dissected into 2-3 mm$^2$ sized pieces using a scalpel. After an additional transfer step as above the tissue pieces were further cut into 1 mm pieces. The pieces finally were transferred into a centrifugation tube filled with 1x PBS and centrifuged for 10 minutes at 1300 rpm. After centrifugation, the supernatant was removed, and the muscle tissue resuspended in 1x PBS supplemented with 8 $\mu$g/ml Gentamicin. The muscle tissue suspension then was cooled to 2-8°C for 48 hours. After the cooling the muscle tissue suspension was centrifuged for 10 minutes at 1300 rpm, the supernatant was then removed and 2.5 ml of a digestion solution containing 1-5 mg/ml collagenase, 2-4 % v/v Hepes buffer, 0.1-10 % v/v fetal calf serum and 5-10 $\mu$g/ml Gentamicin in Ham's F10. The muscle tissue suspension then was incubated for 6 to 20 hours at 37°C, 5% $CO_2$. Next, the suspension was centrifuged at 1300 rpm for 10 minutes, the supernatant was removed, the pellet resuspended in medium containing 10-20% v/v FCS, 1-3 ng/ml bFGF and 3-10 $\mu$g/ml Gentamicin in Hams F10 and plated on cell culture flasks. MPCs attached to the bottom of the culture flask were further maintained by changing medium every 3-4 days and sub cultivation following detachment after confluency was reached. Sub-cultivation was performed until $1 \times 10^6$ to $8 \times 10^7$ MPCs were reached. MPCs isolated according to this example were found to form multinucleated myotubes and highly positive for AChE activity when cultivated in differentiation conditions (Example 2 and 3) (Figure 3 and Figure 5). Further cells were found CD56 and CD90 positive as well as CD34 negative when analyzed according to Example 4 (Figure 6). Further, cells were found to form multinucleated myotubes when cultivated in differentiating conditions according to Example 2 (Figure 3).

Example 2 - Differentiation potential of MPCs

$2 \times 10^6$ MPCs obtained according to Example 1 were seeded in 24-well Nunclon™ Delta Surface plastic plates. About 2 to about 4 days after seeding, skeletal muscle differentiation was initiated in cells by replacing the growth medium with Skeletal Muscle Cell Differentiation medium (500 mL, PromoCell GmbH, Germany), supplemented with 10 mL of Skeletal Muscle Cell Differentiation Medium Supplement Pack (PromoCell GmbH, Germany) and 240 $\mu$L gentamicin (8 mg/mL, Sandoz GmbH, Austria). Cells were cultivated for about 5 to about 7 days and then analyzed by phase contrast microscopy.

MPCs as obtained by Example 1 were tested for *in vitro* differentiation to skeletal myogenic lineage under the appropriate culture conditions. Successful differentiation was found in all MPCs batches used for treatment according to Example 5. Successful myogenic differentiation was determined by microscopic observation of at least one myotube, i.e. a muscle cell with at least 3 separate nuclei formed by fusion of multiple single nucleated MPCs (Figure 3).

Example 3 - AChE enzyme activity measurement

Cells isolated according to Example 1 were analyzed for expression of AChE, following 5-7 days cultivation in differentiation conditions according to Example 2.

*Reagent preparation*

American Public Health Association (APHA) Phosphate buffer, pH 7.2 (Sigma-Aldrich Co. LLC, Germany) was prepared according to manufacturer's instructions. In summary, 17 g of powdered mixture (monopotassium phosphate, 22.66 g/L and sodium carbonate 7.78 g/L) was added into 400 mL distilled water. After adding 0.5 mL Triton X-100, the mixture was dissolved on a magnetic stirrer for 30 minutes at room temperature. The final volume was made up to 500 mL in a measuring cylinder and was used without further dilution. The buffer was stored at 4°C until use. Ellman's reagent (5,5'-dithiobis-2-nitrobenzoic acid, DTNB, 0.5 mM) was prepared freshly for each AChE assay by weighing out 2 mg in 1.5 mL Eppendorf tube. It was dissolved in 1 mL of phosphate buffer (pH 7.2 with 0.1% triton X-100) by vortexing it for 1-2 minutes. The final volume was made up to 10 mL in a 15 mL falcon tube with phosphate buffer (pH 7.2 with 0.1% triton X-100) and was stored at 4°C until use. Acetylthiocholine iodide (ATI, 5.76 mM) was prepared freshly for each AChE assay by weighing out 2 mg in 1.5 mL eppendorf tube. It was dissolved in 1.2 mL of distil water by vortexing for 1-2 minutes and then stored at 4°C until use.

*AChE standard enzyme preparation and measurement:*

AChE standard dilutions were prepared in phosphate buffer (pH 7.2 with 0.1% triton X-100) and were immediately used. A ready to use 50 U/mL AChE stock (from Electrophorus electricus) was purchased from AAT Bioquest® Inc., Sunnyvale, CA, USA. It was diluted to prepare 1000 mU/mL of AChE according to manufacturer's instructions, which was further diluted in a 1:2 ratio to obtain 8 different dilutions ranging from 4-500 mU/mL.

200 $\mu$L of each AChE standard enzyme dilution was mixed with 300 $\mu$L of 0.5 mM DTNB and 50 $\mu$L of 5.76 mM ATI. Additionally, at least one blank reaction mix was created by mixing 0 mU/mL in phosphate buffer with DTNB and ATI as above. Standards and blanks were incubated for 6-, 7- or 8 minutes at 30°C in dark followed by OD measurement at 412 nm on an Anthos Zenyth 340rt microplate reader (Biochrom Ltd., Cambridge, UK). OD value of blank reaction was subtracted from OD values of standard enzyme reactions. Correlation of blank-corrected OD412nm values and AChE concentrations in mU/mL from standard dilutions at was visualized in GraphPad Prism Software. Straight line equation of AChE concentrations and corrected OD values was calculated.

*Measurement of cells:*

In order to measure the AChE activity of cells, cells obtained by cultivation in skeletal muscle differentiation medium according to Example 2 were treated as following: Differentiation medium was carefully removed from 24-well plate with the immediate addition of 300 μL 0.5 mM DTNB solution (prepared in phosphate buffer, pH 7.2 with 0.1% triton X-100). After 2 minutes of incubation at room temperature in dark, 50 μL of 5.76 mM ATI (prepared in distil water) was added. Additionally, at least one blank reaction mix was created by mixing phosphate buffer with DTNB and ATI as above. All reaction mixes were incubated for 60 minutes at 30°C in dark followed by OD measurement at 412 nm on an Anthos Zenyth 340rt microplate reader (Biochrom Ltd., Cambridge, UK).

*Calculation of AChE in $mU_{rel}$:*

OD412nm value obtained as above after 60 min colorimetric measurement of cells were corrected by subtraction of OD412nm values from blank reactions. Corrected OD412nm values of cells at 60 minutes were entered into the straight-line equation generated from AChE standard enzyme reactions (as outlined above with OD at 412 nm measured 6, 7 or 8 minutes after addition of ATI and DTNB) to determine AChE activity per $2*10^5$ cells relative to the AChE standard. Thus, the unit of said determined AChE activity is given in $mU_{rel}$ which refers to a relative AChE activity of $2*10^5$ cells.

MPCs produced according to Example 1 and used for treatment according to Example 5 were found to have at least 36 and up to 680 $mU_{rel}$ AChE per $2*10^5$ cells when measured according to the present Example 3 (Figure 4).

Example 4 - Surface marker expression

Cells isolated according to Example 1 and derived from about 170 distinct subjects were tested for expression of surface markers CD34, CD56 and CD90. To determine surface marker expression, flow cytometry was performed on a Guava easyCyte 6HT 2L flow cytometer (Merck Millipore, Darmstadt, Germany). Briefly, cells obtained by Example 1 were harvested by covering with 1X trypsin at 37°C for 5 minutes, centrifuged at 400*g and resuspended in 1X PBS supplemented with 1% FCS. 40 000 cells were resuspended in 195 μl 1X PBS and incubated after addition of 5 μ L CD34-PE, CD56-PE, CD90-PE, (Beckman Coulter) for 20 minutes in a 1.5 mL Eppendorf tube at 4°C in dark. Then, each reaction received 5 μ L of viability dye 7-aminoactinomycin D (Beckman Coulter Inc., France) and the plate was incubated for 10 minutes at RT in dark. Finally, cell events were acquired with Guava InCyte™ v.2.3 software. Histograms and dot-plots were generated with a minimum of 5000 events at a sample flow rate of 1.8 μL/mL. Positive staining was obtained by comparison with Isotype control set as at least 95% negative or comparison to control (negative) cells.

Further, individual randomly and exemplarily selected batches of the cells obtained by Example 1 were tested for Sca-1, A2B5 and CD105 expression by Flow Cytometry. Therefore, flow cytometry analysis was performed on a Guava easyCyte 6HT 2L flow cytometer (Merck Millipore, Darmstadt, Germany). Briefly, cells were harvested by trypsin at 37°C for 5 min, centrifuged at 400 rcf and resuspended in 1x PBS supplemented with 1% FCS. Cells in a concentration of 40000/reaction were incubated with 5 μL Isotype IgG1-PE (Beckman Coulter), Isotype Alexa488 (Sigma), anti-CD105-PE (Beckman Coulter Inc., France) or A2B5-Alexa488 antibody (Millipore) for 15 min in a 1.5 mL Eppendorf® tube at 4° C in dark. Cells were washed with 1 mL PBS, centrifuged at 400 rcf and resuspended in 200 μL of 1x PBS for FACS analysis in a 96 well round bottom plate. After washing and resuspension, each reaction received 5 μL of viability dye 7-aminoactinomycin D (Beckman Coulter Inc., France) and plate was incubated for 10 minutes at 4° C. Cell events were acquired with the help of Guava InCyte™ v.2.3 software. Histograms and dot-plots were generated with a minimum of 3000 events with a sample flow rate of 1.8 μL/mL. Positive staining was obtained by comparison with Isotype control set as at least 99% negative.

MPCs batches produced according to Example 1 and used for treatment according to Example 5 were found to be positive for CD56 in the range of 64.10 % to 99.83 %, positive for CD90 in the range of 80.05 % to 99.88 % and positive for CD34 in the range of 0.04 % to 8.33 %. This expression profile was determined over MPCs batches isolated from biopsies of about 170 distinct patients. Further, individual randomly and exemplarily selected batches of said MPCs batches were found to be positive for A2B5 and CD105 at least by positive expression of 60% cells per batch for each marker and found to be Sca-1 negative, wherein at maximum 10 % of cells were positive for Sca-1.

For intracellular detection of desmin, and MyoD expression in cells isolated according to Example 1, immunocytochemistry was performed. First, supernatant of cell culture dishes was discarded, and cells were washed three times with PBS. Permeabilization and fixation was carried out by covering the cells with 4% formaldehyde solution (v/v; diluted in PBS) for 20 minutes at room temperature. Then, the cells were washed with PBS three times after each conducted incubation step. Afterwards, cells were covered with 500 μl hydrogenperoxid-block (Thermo Fisher Scientific) and have been incubated for five minutes at room temperature. Primary antibodies (desmin, or MyoD) in a final concentration of 40 μg pro ml (w/v) were pipetted on the cells and have been incubated for at least 90 minutes (37°C, 5% CO$_2$), Cells were then covered with 500 μl biotin-conjugated secondary antibodies (goat anti rabbit, polyclonal, Thermo Fisher Scientific) and have been incubated under the same conditions as for primary antibodies but 60 minutes at least. For visualization of antibody bindings, 500 μl of horseradish streptavidin peroxidase (Vectorlabs) had to be added in a final concentration of 2-5 μg/ml (diluted in PBS) and incubated at 37° C, 5% CO2, 20 minutes long followed by covering the cells with 500 μl chromogen single-solutions, which was removed after 5 to 15 minutes. A final washing step with PBS was carried out before results could be observed. Cells stained desmin positive by immunocytochemistry are visualized in dark red color.

Individual randomly and exemplarily selected batches of the MPCs batches isolated according to Example 1 as outlined above were found to be Desmin positive, wherein at least 60% of cells were found positive. Further, individual randomly and exemplarily selected batches of the MPCs batches isolated according to Example 1 were found to be MyoD negative, wherein at maximum 10% of cells were found positive for MyoD.

Example 5 - Treatment of fecal incontinent patients using MPCs

The applicant conducted a multinational, multicenter, randomized, double-blinded, placebo-controlled parallel-group clinical phase IIb study as a proof-of-concept trial investigating the efficacy of myogenic progenitor cell-based treatment in a patient population reflecting the real-life diversity encountered in clinical practice. Patient inclusion and exclusion criteria are listed below (Table 1). An overview of conducted visits and investigations as well as patient flow is visible in Figure 4.

*Table 1: Inclusion and Exclusion Criteria for patients to be included into the ITT population of the clinical trial according to Example 5.*

| Inclusion Criteria | Exclusion Criteria |
|---|---|
| Patient ≥18 years of age, | Patient with pathological findings considered clinically relevant by the investigator (excluding sphincter damage) based on rectoscopy, anorectal manometry and ultrasound at the screening visit (e.g. grade I haemorrhoids were considered acceptable) |
| Patient suffering from fecal incontinence for more than 6 months, which is confirmed at screening by relevant medical history and anorectal examination. | Patient who had undergone any anorectal surgery within the last 6 months prior to screening visit |
| Patient with Wexner score >9 and with at least 3 episodes of fecal incontinence per week as measured in the bowel diary prior to Visit -1. | Patient with more than one overlap repair surgery |
| Patient with no indications against a surgery under anesthesia. | Patient with more than 2 anorectal surgical procedures in total (e.g. primary repair after delivery and one overlap repair later-on or in- and explantation of a permanent neurostimulation system) |
| Patient willing and able to comply with the study procedures. | Patient with overlap repair and associated early atrophy of external anal sphincter |
| Patient who is mentally competent and able to understand all study requirements. | Patient with a history of artificial anal sphincter (AAS) surgery |
| Patient who agrees to read and sign the informed consent form prior to any study-related procedures. | Patient with trans- or perianal injection of any bulking products |

(continued)

| Inclusion Criteria | Exclusion Criteria |
|---|---|
| Patient willing to use acceptable methods of contraception (birth control pills, barriers, or abstinence) [For female patients of childbearing potential]. | Patient with a malignant disease not in remission for 5 years or more |
| Patient with a minimum of 3 non-gaseous incontinence episodes per week measured using the diary during the 2-week period prior to Visit-1 (day -70) | Patient who had undergone radiation therapy of the bowel and pelvis |
| | Patient who had undergone chemotherapy within last 5 years prior to study enrolment |
| | Patient with chemotherapy related neuropathy of the bowel and pelvis |
| | Patient with compromised immune system and/or rheumatic disease |
| | Patient under immunosuppressive therapy |
| | Patient with a diagnosis of chronic inflammatory bowel disease |
| | Patient with current and/or recurrent anal fistula disease |
| | Patient with chronic diarrhea |
| | Patient suffering from a disease which has not been resolved within a timeframe prior to screening as follows: fever and/or diarrhea of unknown reasons (4 weeks), HAV (4 months), toxoplasmosis (6 months), osteomyelitis, Q fever, rheumatic fever, tuberculosis, or Salmonella infections (2 years), and malaria (4 years) |
| | Patient who, according to the clinical judgment of the investigator, is not suitable for inclusion due to acute anal sphincter injury including obstetric and other trauma, acute disc prolapses, or neurological diseases (spinal cord injury, multiple sclerosis, Parkinson's disease, stroke, etc.) |
| | Patient with uncontrolled diabetes mellitus type I or II, or suffering from diabetic peripheral neuropathic pain |
| | Patient diagnosed with human immunodeficiency virus (HIV), acute or chronic viral hepatitis HCV, acute or chronic viral hepatitis HBV, active Syphilis, HTLV (tested upon risk assessment by investigator) |
| | Patient diagnosed with any kind of skeletal muscle disease and/or neuronal disorders |
| | Patient with known hypersensitivity to any component of the product (autologous cells, ringer's lactate, human serum albumin, DMSO, bovine proteins, fibroblast growth factor) |
| | Patient with clinically relevant abnormal laboratory values, any persistent chronic bacterial infections as well as local infections as indicated by a high level of the C-reactive protein (i.e. >35 mg/L) and confirmed by bacteriological analysis, or with any bleeding disorder |
| | Patient who, according to the clinical judgment of the investigator, is not suitable for this study |
| | Patient who is currently participating or has participated in another clinical trial (testing a medical device or drug) within 30 days prior to the study begin or has previously participated in the current clinical study |

(continued)

| Inclusion Criteria | Exclusion Criteria |
|---|---|
| | Patient who is pregnant, lactating, or intending pregnancy in the near future (before Visit 4), and of childbearing potential who is not willing to use acceptable methods of contraception up to Visit 4 (birth control pills, barriers, or abstinence) or who has a positive pregnancy test (only to be performed in women of childbearing potential) |
| | Patient dependent from the Sponsor, CRO, or the investigator (e.g., employees, relatives, etc.) |
| | Patient deprived of his/her liberty by a judicial or administrative decision, patient admitted to a hospital, social institution or who is under a measure of legal protection, patient hospitalized without consent or who is in an emergency situation |
| | Patient with severe myocardial disorders, irregular pulse or a pacemaker |
| | Patient with implantations of metal components in the electrical stimulation treatment area |
| | Patient with chronic constipation and/or overflow incontinence |
| | Patient with persistent bacterial infection(s) confirmed by clinical signs and positive results in bacteriological testing at Visit-1 (Salmonella (Typhus), F. tularensis (Tularaemia), M. leprae (Leprosy), Brucella, Rickettsia). Bacteriological analysis had to be performed for patients with an elevated level of the CRP (i.e. >35 mg/L). CRP level and results of the bacteriological analyses had to be checked at Visit 0 to ensure the patients had no bacteriological infections. |

Eligible patients were randomized into one of three treatment arms (for the randomization procedure see supplementary methods) and received either cell therapy with a low (LCC, $5 \pm 1*10^6$) or a high dose (HCC, $50 \pm 10*10^6$) of injected cells isolated according to Example 1 in combination with 4 weeks of electrical stimulation or were subjected to a control treatment with injection of cell free medium combined with the electrical stimulation. MPCs implantation was conducted in anaesthetized patients as described previously by a trained physician. Patients were placed in a supine position and cells or placebo were injected under direct ultrasound guidance using a specifically designed injection device. Each patient received an aliquot of frozen cells diluted to the appropriate cell concentration with Ringer's lactate. The resulting total volume of 6 ml was administered in 12 depots ($12 \times 0.5$ ml), each of which was extended in a circular array directly into the external anal sphincter (EAS). Injection of cells into the longitudinal muscle, internal anal sphincter or sub epithelium was avoided. For the procedure, all patients were hospitalized for 1 day. Cells isolated according to Example 1 and used for implantation were analyzed for skeletal muscle differentiation potential (Example 2), AChE enzyme activity (Example 3) and surface marker expression (Example 4). The cell batches produced during the trial were all found positive for potential to form multinucleated myotubes (Figure 3). Further, cell populations used for both LCC and HCC treatment were found to be 64.10 % to 99.83 % positive for CD56 and 80.05 % to 89.99 % positive for CD90 as well as 0.04 % to 8.33 % positive for CD34 (Figure 6). Pelvic floor electrical stimulation therapy is considered a gold standard for conservative treatment of fecal incontinence as it is known to stimulate muscle growth and growth associated signaling. The study overview is outlined in Figure 4A. Patients selected for treatment within the trial (intention to treat population, ITT) were having the basic demographic parameters as shown in Table 2.

*Table 2: Demographics, time since first diagnosis of FI and associated sphincter abnormalities in ITT patient population of the clinical trial*

| | | PBO N=79 | LCC N=83 | HCC N=75 | Total N=237 |
|---|---|---|---|---|---|
| Age, *years* | | | | | |
| | Mean ± SD | 59.4 ± 14.8 | 59.6 ± 13.2 | 61.4 ± 13.7 | 60.1 ± 13.9 |
| | Median | 63.0 | 61.0 | 64.0 | 63.0 |
| | Q1; Q3 | 52.7 ; 71.0 | 54.0 ; 69.0 | 54.0 ; 70.5 | 53.8 ; 70.0 |
| | Min ; Max | 23 ; 85 | 21 ; 83 | 26 ; 85 | 21 ; 85 |

(continued)

| | | PBO | LCC | HCC | Total |
|---|---|---|---|---|---|
| Gender, *female* | | | | | |
| | n (%) | 73 (92.4%) | 75 (90.4%) | 70 (93.3%) | 218 (92.0%) |
| Country | | | | | |
| Austria | n (%) | 24 (30.4%) | 25 (30.1%) | 25 (33.3%) | 74 (31.2%) |
| Bulgaria | n (%) | 4 (5.1%) | 5 (6.0%) | 3 (4.0%) | 12 (5.1%) |
| Czech Republic | n (%) | 11 (13.9%) | 8 (9.6%) | 8 (10.7%) | 27 (11.4%) |
| France | n (%) | 1 (1.3%) | 1 (1.2%) | 0 (0.0%) | 2 (0.8%) |
| Germany | n (%) | 23 (29.1%) | 23 (27.7%) | 23 (30.7%) | 69 (29.1%) |
| Slovenia | n (%) | 3 (3.8%) | 3 (3.6%) | 2 (2.7%) | 8 (3.4%) |
| Sweden | n (%) | 12 (15.2%) | 16 (19.3%) | 12 (16.0%) | 40 (16.9%) |
| Switzerland | n (%) | 0 (0.0%) | 0 (0.0%) | 1 (1.3%) | 1 (0.4%) |
| United Kingdom | n (%) | 1 (1.3%) | 2 (2.4%) | 1 (1.3%) | 4 (1.7%) |
| BMI, *kg/m²* | | | | | |
| | Mean ± SD | 24.7 ± 4.6 | 25.9 ± 4.4 | 26.0 ± 4.7 | 25.6 ± 4.6 |
| | Median | 24.2 | 25.4 | 25.2 | 25.1 |
| | Q1 ; Q3 | 20.8 ; 27.5 | 22.7 ; 28.3 | 22.7 ; 28.2 | 22.2 ; 27.9 |
| | Min ; Max | 17 ; 41 | 16 ; 37 | 19 ; 41 | 16 ; 41 |
| Race | | | | | |
| Caucasian | n (%) | 78 (98.7%) | 82 (98.8%) | 75 (100.0%) | 235 (99.2%) |
| Asian | n (%) | 0 (0.0%) | 0 (0.0%) | 0 (0.0%) | 0 (0.0%) |
| | | **PBO N=79** | **LCC N=83** | **HCC N=75** | **Total N=237** |
| African | n (%) | 0 (0.0%) | 0 (0.0%) | 0 (0.0%) | 0 (0.0%) |
| NA [1] | n (%) | 1 (1.3%) | 1 (1.2%) | 0 (0.0%) | 2 (0.8%) |
| Time since first diagnosis of FI, *years* | | | | | |
| | Mean ± SD | 7.9 ± 8.6 | 6.7 ± 5.8 | 7.8 ± 7.1 | 7.5 ± 7.2 |
| | Median | 5.5 | 4.9 | 5.3 | 5.3 |
| | Q1 ; Q3 | 2.5 ; 9.1 | 2.9 ; 8.8 | 2.7 ; 11.1 | 2.7 ; 9.9 |
| | Min ; Max | 1 ; 46 | 0 ; 31 | 1 ; 33 | 0 ; 46 |
| Conditions associated with incontinence | | | | | |
| Diarrhea | n (%) | 1 (1.3%) | 6 (7.2%) | 1 (1.3%) | 8 (3.4%) |
| Constipation | n (%) | 0 (0.0%) | 0 (0.0%) | 0 (0.0%) | 0 (0.0%) |
| Muscle damage | n (%) | 40 (50.6%) | 42 (50.6%) | 41 (54.7%) | 123 (51.9%) |
| Pelvic floor dysfunction | n (%) | 10 (12.7%) | 10 (12.0%) | 12 (16.0%) | 32 (13.5%) |
| Nerve damage | n (%) | 2 (2.5%) | 5 (6.0%) | 1 (1.3%) | 8 (3.4%) |
| Loss of storage capacity | n (%) | 0 (0.0%) | 1 (1.2%) | 0 (0.0%) | 1 (0.4%) |
| Atrophy | n (%) | 28 (35.4%) | 28 (33.7%) | 23 (30.7%) | 79 (33.3%) |
| Other | n (%) | 2 (2.5%) | 1 (1.2%) | 2 (2.7%) | 5 (2.1%) |
| Type of FI | | | | | |
| Passive incontinence | n (%) | 20 (25.3%) | 25 (30.1%) | 15 (20.0%) | 60 (25.3%) |
| Urge incontinence | n (%) | 56 (70.9%) | 54 (65.1%) | 58 (77.3%) | 168 (70.9%) |
| Fecal seepage | n (%) | 3 (3.8%) | 4 (4.8%) | 2 (2.7%) | 9 (3.8%) |
| History of anorectal surgery | n (%) | 17 (21.5%) | 15 (18.1%) | 15 (20.0%) | 47 (19.8%) |
| | | **PBO N=79** | **LCC N=83** | **HCC N=75** | **Total N=237** |
| History of AAS surgery | n (%) | 0 (0.0%) | 0 (0.0%) | 0 (0.0%) | 0 (0.0%) |

(continued)

|  | PBO N=79 | LCC N=83 | HCC N=75 | Total N=237 |
|---|---|---|---|---|
| History of AAS | | | | |
| [1] Race not collected, as per French legislation | | | | |
| BMI, body mass index; HCC, high cell count; ITT, intention-to-treat; LCC, low cell count; PBO, placebo; Q1, first quartile, Q3; third quartile; SD, standard deviation | | | | |

Example 6 - Efficacy Analysis

Following the initial screening visit of Example 5, patients were instructed to fill an incontinence diary for two weeks before a muscle biopsy sample was removed from the *Musculus pectoralis major* on their next visit. Subsequently a four-week period of electrical stimulation treatment was initiated. After another four weeks of filling a diary, patients received their cell (LCC or HCC) or control (PBO) injection, underwent a post implantation control on the next day. Patients received yet another four-week period of electrical stimulation treatment paralleled by diary keeping. At three, six-, and 12-months post injection patients were retrieved for control visits which were preceded by four weeks of diary keeping. In the diary, patients were requested to daily score how fecal incontinence negatively affected their day on a visual analogue scale (VAS), and to keep track of FI episodes and to classify these into three categories referred to as "traces", "little" and "more". Further parameters were acquired as outlined in the scheme and comprised assessment of the responder rates, anorectal manometry, and ultrasound measurements, and a FI-QoL questionnaire. As the primary endpoint we defined the change in incontinence episode frequency (IEF) assessed for the four-week period before the 6 months post-injection visit (V4 = 6 months post treatment in Figure 1) compared to the baseline value determined for the four-week period before injection (V0). Secondary endpoints that were assessed comprised changes in VAS, calculated from the mean of the four-week diary period, and QoL. In addition, we assessed the share of patients responding with more than 25%, 50%, 75% and 90% reduction in IEF compared to baseline, changes in the anorectal manometry and sonography data over time. Further exploratory endpoints were changes in IEF and any of these other parameters from baseline to 12 months post-injection, alterations in different types of incontinence episodes (IE) and incontinence free days.

Anal endoscopy, ultrasound examination and anorectal manometry measurement were performed according to standards set in each of the participating centres. Parameters assessed included length of the anal canal, resting pressure, and maximal squeeze pressure. Balloon expulsion tests were used to measure the filling volume until the first sensation was reached, the volume for desire to defecate, the volume for urgency for defecation, and the maximal tolerable volume.

Throughout the study the occurrence of adverse events (AE) and serious AE was recorded, physical examinations and standard tests of haematology, blood chemistry, and urinalysis were conducted, and the concomitant medication registered. The study was overseen by an independent data safety monitoring board. This estimate yielded a total of 252 patients to be randomized in a 1:1:1 ratio to the three treatment arms. For primary endpoint analysis we applied a one-sided Wilcoxon rank-sum test considering a p value < 0.025 as significant. Secondary endpoints of continuous variables were compared between study arms by unpaired t-test (if compared study arms were normally distributed) or Wilcoxon rank-sum test (if compared study arms were not normally distributed), considering a p < 0.05 as significant. Non-continuous variables were compared between study arms by Chi-squared or Fisher test.

Analysing the treatment efficacy in terms of 50% responder rate of PBO, LCC and HCC treatment on different types of incontinence episodes on visit 5 (12 months post treatment) revealed that responder rates were consistently lowest for episodes classified as "traces" (Table 3). Thus, for further analyses of IEF change and responder rates, calculations were made for either counting all episode types or counting only episode types "little" and "more".

*Table 3: Percent of patients having at least 50% reduction of weekly incontinence episodes from baseline to 12 months post treatment by treatment group (LCC, HCC, PBO) and episode type (traces, little, more.*

|  | **"Traces"** | **"Little"** | **"More"** |
|---|---|---|---|
| **LCC** | 37.1 | 38.7 | 45.8 |
| **HCC** | 32.1 | 55.6 | 33.3 |
| **PBO** | 22.2 | 29.4 | 42.1 |

Analysis of changes in IEF among treatment groups in ITT patient population revealed a consistent reduction over the post implantation visits in all treatment groups independent if all episode types were counted or traces were excluded (Figure 1A, Figure 1B, Table 4 and Table 5). Highest reduction in IEF was found at all post treatment visits in HCC group followed by LCC and finally PBO group. Conducting 2-sided Wilcoxon Mann-Whitney tests at an alpha level of 0.05 between HCC vs. PBO and LCC vs. PBO in the ITT set, it was found that change in IEF from baseline to post implantation visits was significantly increased in HCC compared to PBO at 6 months post treatment (p=0.035) when traces were included and at 12 months (p=0.034) when traces were excluded (Figure 1A and B), suggesting that high cell count implantation of MPCs is superior to placebo treatment.

*Table 4: Absolute change in weekly IEF (including traces) from baseline (V0) over the study period of Example 5 according to treatment arm in the ITT patient population. PBO, Placebo. LCC, low cell count. HCC, high cell count. SD, standard deviation. N, number of patients.*

| | PBO | | | LCC | | | HCC | | |
|---|---|---|---|---|---|---|---|---|---|
| **Months post TREATMENT** | *Mean* | *SD* | *N* | *Mean* | *SD* | *N* | *Mean* | *SD* | *N* |
| **1** | -1.9 | 3.8 | 79 | -3.4 | 6 | 83 | -3.8 | 6.9 | 75 |
| **3** | -2.7 | 4.8 | 79 | -4.3 | 7.3 | 83 | -4.5 | 6.9 | 75 |
| **6** | -3.2 | 5.2 | 79 | -4.3 | 8.2 | 83 | -4.8 | 6.8 | 75 |
| **12** | -2.5 | 4.9 | 79 | -4.4 | 7.3 | 83 | -5.1 | 9.4 | 75 |

*Table 5: Absolute change in weekly IEF (excluding traces) from baseline (V0) over the study period of Example 5 according to treatment arm in the ITT patient population. PBO, Placebo. LCC, low cell count. HCC, high cell count. SD, standard deviation. N, number of patients.*

| **Months post TREATMENT** | PBO | | | LCC | | | HCC | | |
|---|---|---|---|---|---|---|---|---|---|
| | *Mean* | *SD* | *N* | *Mean* | *SD* | *N* | *Mean* | *SD* | *N* |
| **1** | 1 | -1.4 | 3.2 | 79 | -2 | 4.6 | 83 | -2.4 | 5.1 |
| **3** | 3 | -2.3 | 5.2 | 79 | -3 | 5.8 | 83 | -3.3 | 5.2 |
| **6** | 6 | -2.7 | 5 | 79 | -2.9 | 6.2 | 83 | -3.7 | 5.2 |
| **12** | 12 | -1.7 | 4.2 | 79 | -3 | 5.6 | 83 | -3.7 | 6.3 |

*Responder rates*

In agreement with others (Rao, 2016), we considered a reduction of IEF by $\geq$ 50% a clinically relevant improvement and classified patients showing this extent of reduction as responders, distinct from non-responders with a smaller reduction. Accordingly, we evaluated our data regarding the share of responders in each treatment arm, including data from one- and three-months post-injection, and revisiting the subgroups specified above. We detected that in all groups and sub-populations defined the share of responders continuously increased up to the six-months follow-up, with the cell groups exceeding the controls in all cases, and HCC exceeding LCC in most cases (Percent of patients with at least 50% reduction in weekly IEF (including traces) from baseline to post treatment visits according to Example 5 by treatment groups in the ITT patient population. PBO, Placebo. LCC, low cell count. HCC, high cell count (Table 6). Between six and 12 months the responder share of controls and LCC either plateaued or even declined, whereas it continued to increase up to more than 50% in the HCC group, when including traces (Table 6), and more than 60%, when excluding traces (Table 7). Conducting Fisher's exact tests on 50% responder rates with a significance margin at p=0.05 between LCC or HCC and PBO treatment in ITT patients, a superiority of HCC over PBO treatment was found at 1 (p=0.037) and 12 months (p=0.006) post treatment when traces were excluded from the analysis (Figure 1D, Table 7). This suggests that high cell count application of myogenic progenitor is an effective and clinically relevant treatment for incontinence.

*Table 6: Percent of patients with at least 50% reduction in weekly IEF (including traces) from baseline to post treatment visits according to Example 5 by treatment groups in the ITT patient population. PBO, Placebo. LCC, low cell count. HCC, high cell count.*

| **Months post TREATMENT** | **PBO** | **LCC** | **HCC** |
|---|---|---|---|
| **1** | 16.9 | 26.8 | 27 |
| **3** | 36.4 | 42.7 | 45.9 |
| **6** | 44.2 | 50 | 50 |

(continued)

| Months post TREATMENT | PBO | LCC | HCC |
|---|---|---|---|
| 12 | 40.3 | 47.6 | 54.1 |

*Table 7: Percent of patients with at least 50% reduction in weekly IEF (excluding traces) from baseline to post treatment visits according to Example 5 by treatment groups in the ITT patient population. PBO, Placebo. LCC, low cell count. HCC, high cell count*

| Months post treatment | PBO | LCC | HCC |
|---|---|---|---|
| 1 | 28.4 | 42.3 | 45.1 |
| 3 | 47.3 | 56.4 | 59.2 |
| 6 | 51.4 | 60.3 | 64.8 |
| 12 | 43.2 | 56.4 | 66.2 |

Example 7 - Patient characteristics and relation to treatment outcome

Within the clinical trial outlined in Example 5, 288 patients were screened, 251 were randomized, of which 244 received study medication. 218 women and 19 men completed the study at least up to the six months follow-up (96%) (Figure 4). The median age of the participants was 63 years (inter quartile range, IQR, 53.8 - 70) and they had suffered from FI for a median period of 5.3 years (IQR 2.7 - 9.9). The baseline demographic and clinical characteristics of ITT patients available for primary endpoint analysis are summarized in Table 2, which also shows that the assignment to different treatment groups was well balanced.

*Subgroup-specific impact*

To establish a patient group, specifically responsive to the cell treatment rather than to the active placebo, we conducted explorative *post-hoc* analyses. To this end, we applied the following hypothesis-driven approach. We hypothesized that for the injected cells to restore EAS function, it is useful to be injected into or near existing muscle tissue. This could be due to limited migratory capacities of MPCs following implantation. Muscle regeneration may thus have been impaired in patients where muscle tissue was rare due to persistent scar formation of damaged EAS or because of time-dependent sarcopenia. Both conditions will correlate with FI duration prior to the study. Re-analysing the treatment effect in only those patients suffering from FI for ≤ 10 years (73% of the ITT set; target population 1, TPP1), the decline of IEF in the LCC and the HCC group significantly exceeded that in the controls ultimately resulting in a much higher change in IEF from baseline to 12 months when compared to the respective changes in the ITT set when excluding traces (Figure 2B). Regarding responder rates and within TPP1 patient subgroup, a significantly higher 50% responder rate was found for HCC than PBO at 12 months post treatment when traces were excluded (Figure 2B).

Another factor diminishing the apparent impact of cell treatment was that IEF also declined in the controls. Therefore, to clearly capture the effect of cell injection, baseline IEF we hypothesized that higher baseline IEF would be beneficial to see higher IEF reduction post treatment and clear difference between PBO and cell groups (LCC, HCC). This hypothesis was tested by subgrouping ITT set patients according to their baseline IEF (including traces) and comparing change in IEF from baseline to 6 months among treatment groups and subgroups. It was found that subsequential exclusion of lower baseline IEF patients lead to an increase in IEF change in all groups. However, differences between PBO group and cell groups appeared increasingly prominent with increasing baseline IEF (Figure 7, Table 8). This suggests that patient with higher baseline IEF benefit more from MPCs-based treatment compared to low baseline IEF patients. As starting from >6 IEF baseline, differences between treatment groups were rapidly increasing, we combined TPP1 with this characteristic to form TPP2 (≤10 years' time since first diagnosis of FI and >6 IEF at baseline). Re-analysing the treatment effect in only those patients the decline of IEF in the LCC and the HCC group significantly exceeded that in the controls ultimately resulting in a much higher change in IEF from baseline to 12 months when compared to the respective changes in the ITT and TPP1 (Figure 2A). Regarding responder rates of TPP2 patient subgroup, a significantly higher 50% responder rate was found for HCC than PBO at 12 months post treatment ultimately leading to higher responder rates in HCC group of TPP2 compared to HCC group of ITT patients (Figure 2C). This suggests that TPP2 patients are more responsive to MPCs-based treatment compared to ITT patients.

As it was demonstrated above that incontinence episodes classified as traces are least responsive to the treatment according to Example 5, and that higher baseline IEF patient respond better to MPCs-based treatment, we analysed TPP1 patients having more than 2 baseline IEF that may not be classified as "traces". This patient population was called TPP3. Re-analysing the treatment effect in only those patients of TPP3 the decline of IEF in the LCC and the HCC group significantly exceeded that in the controls, when traces were not counted (Figure 2B), ultimately resulting in a much higher change in IEF from baseline to 12 months when compared to the respective changes in the ITT, TPP1 and TPP2 (Figure 2A and Figure 2B). Regarding responder rates of TPP3 patient subgroup, a significantly higher 50% responder rate was found for HCC than PBO at 12 months post treatment when traces were included. Not including traces in the analysis, a significantly higher responder rate was found for both HCC and LCC compared to PBO. Ultimately, TPP3 responder rates of HCC treated patients were the highest compared to all other patient groups (Figure 2C and Figure 2D) independent of counting traces as episodes or not (Table 11, Table 12). This suggests that TPP3 patients are most responsive to MPCs-based treatment compared to ITT, TPP1 and TPP2 patients. Vice versa, patients excluded by TPP3 subgrouping (i.e. having >10 years of FI duration termed "NR1", or less than 2 IEF at baseline (excluding traces) termed "NR2" were having lower IEF changes and responder rates than TPP1, TPP2 and TPP3 patients. Selection of patient groups might not only have an impact on how cell therapeutic treatment by LCC or HCC performs on IEF reduction and response rate, but also have an impact on how placebo (PBO) treatment performs. Thus, we further calculated effect sizes and odd's ratios for IEF change and responder rates, respectively. In detail, Cohen's d and odd's ratio was calculated comparing either LCC or HCC with PBO treatment of patient subgroups according to Table 13. Independent of counting traces as episodes or not, TPP3 patients had the highest treatment effect by LCC or HCC treatment (each compared to PBO treatment) in terms of effect size and odd ratio (Figure 8).

We further analysed IEF change and responder rate from baseline to 12 months post treatment as well as effect sizes and odd ratios associated with it in all TPP3 patients compared to TPP3 patients having an external anal sphincter muscle damage (TPP3_damage) associated with their faecal incontinence before treatment (Table 9, Table 10, Table 11, Table 12). Comparing these patient groups, we found that higher IEF reduction following HCC treatment was found in TPP3 patients suffering from FI due to muscle damage compared to TPP3 patients with unspecified cause of FI (Figure 9 A and B). This effect was found independent of counting traces as FI or not. Also, in TPP3_damage population, IEF decrease following HCC treatment was significantly higher compared to PBO treatment. Similarly, it was found that 50% responder rate was higher in TPP3_damage population independent of counting or excluding traces (Figure 9 C and D). Significantly higher responder rates following HCC than PBO treatment were found in TPP3_damage patients when traces were excluded from the analysis ultimately reaching an all-time high responder rate of 87.5 % in HCC treated patients (Table 12). Analysing effect sizes and odds ratios of IEF change and responder rates by LCC or HCC compared to PBO treatment in TPP3_damage patients, revealed higher effect sizes of HCC treatment in TPP3_damage than TPP3 patient population independent of accounting traces as incontinence episodes or not (Figure 10 A, B). Odds ratios of 50% responder rates were also found higher in TPP3_damage patients compared to TPP3 patient population for both LCC and HCC treatment, each independent of counting or excluding traces (Figure 10 C, D). These results suggest that patients suffering from EAS damage prior to treatment are experiencing higher reduction in incontinence episodes and higher change for response when treated with different numbers of MPCs compared to the broader patient population with multiple possibilities of FI associated conditions (e.g. EAS atrophy, pelvic floor dysfunction, etc.).

*Table 8: Change in IEF from baseline to 6 months post treatment in ITT set further grouped according to baseline IEF among treatment groups (PBO, LCC, HCC). Mean, standard deviation "SD" and number of patients "N" is demonstrated.*

| Baseline IEF | PBO | | | LCC | | | HCC | | |
|---|---|---|---|---|---|---|---|---|---|
| | *Mean* | *SD* | *N* | *Mean* | *SD* | *N* | *Mean* | *SD* | *N* |
| >0 | -3.2 | 5.2 | 77 | -4.3 | 8.3 | 82 | -4.6 | 6.7 | 74 |
| >1 | -3.4 | 5.2 | 76 | -4.3 | 8.3 | 82 | -4.6 | 6.7 | 74 |
| >2 | -3.3 | 5.2 | 74 | -4.4 | 8.4 | 80 | -4.7 | 6.7 | 72 |
| >3 | -3.5 | 5.4 | 69 | -4.6 | 8.6 | 76 | -4.9 | 6.8 | 68 |
| >4 | -3.6 | 5.5 | 65 | -5 | 8.7 | 71 | -5 | 7 | 65 |
| >5 | -3.7 | 5.9 | 57 | -5.6 | 9.5 | 58 | -5.2 | 7.2 | 60 |
| >6 | -3.9 | 6.3 | 47 | -5.9 | 9.6 | 54 | -6 | 7.6 | 53 |
| >7 | -4.2 | 6.5 | 43 | -6.5 | 10 | 47 | -5.8 | 7.3 | 48 |
| >8 | -4.5 | 6.8 | 37 | -7.3 | 11 | 40 | -7.2 | 8 | 37 |
| >9 | -4.7 | 7 | 34 | -7.7 | 11 | 37 | -7.7 | 8.4 | 32 |
| >10 | -5.4 | 7.1 | 30 | -9 | 11 | 32 | -8.8 | 7.5 | 29 |

(continued)

| Baseline IEF | PBO | | | LCC | | | HCC | | |
|---|---|---|---|---|---|---|---|---|---|
| | *Mean* | *SD* | *N* | *Mean* | *SD* | *N* | *Mean* | *SD* | *N* |
| >11 | -5.4 | 7.6 | 25 | -10 | 12 | 26 | -9 | 7.6 | 28 |
| >12 | -6 | 7.9 | 22 | -11 | 12 | 24 | -9.1 | 8.5 | 22 |

*Table 9: Change in IEF (including traces) from baseline to 12 months post treatment according to treatment groups (PBO, LCC, HCC) and patient populations demonstrated by mean, standard deviation (SD) and patient number (N).*

| | ITT | | | TPP1 | | | TPP2 | | | TPP3 | | | NR1 | | | NR2 | | | TPP3_damage | | |
|---|---|---|---|---|---|---|---|---|---|---|---|---|---|---|---|---|---|---|---|---|---|
| | *Mean* | *SD* | *N* | *Mean* | *SD* | *N* | *Mean* | *SD* | *N* | *Mean* | *SD* | *N* | *Mean* | *SD* | *N* | *Mean* | *SD* | *N* | *Mean* | *SD* | *N* |
| PBO | -2.6 | 5.1 | 79 | -2.8 | 4.8 | 45 | -2.8 | 5.8 | 38 | -2.5 | 5.6 | 23 | -3.5 | 5.6 | 16 | -2.4 | 4.7 | 50 | -2.4 | 5.6 | 12 |
| LCC | -3.8 | 6.6 | 83 | -5.3 | 8.4 | 48 | -6 | 9.1 | 44 | -6.6 | 8.9 | 24 | -2.7 | 5.8 | 17 | -3.3 | 6.4 | 52 | -4.9 | 6.2 | 9 |
| HCC | -5.5 | 10.9 | 75 | -5.3 | 8 | 39 | -7.1 | 7.7 | 37 | -9.1 | 9.9 | 18 | -4.2 | 13.1 | 24 | -3.4 | 4.9 | 47 | -8.6 | 5.4 | 9 |

Table 10: Change in IEF (excluding traces) from baseline to 12 months post treatment according to treatment groups (PBO, LCC, HCC) and patient populations demonstrated by mean, standard deviation (SD) and patient number (N).

| | ITT | | | TPP1 | | | TPP2 | | | TPP3 | | | NR1 | | | NR2 | | | TPP3_damage | | |
|---|---|---|---|---|---|---|---|---|---|---|---|---|---|---|---|---|---|---|---|---|---|
| | Mean | SD | N | Mean | SD | N | Mean | SD | N | Mean | SD | N | Mean | SD | N | Mean | SD | N | Mean | SD | N |
| PBO | -1.7 | 4.2 | 79 | -2.3 | 4.8 | 63 | -2.6 | 6 | 24 | -2.6 | 5.3 | 23 | -1.3 | 3.8 | 50 | -1.7 | 4.2 | 79 | -2.0 | 4.4 | 12 |
| LCC | -3 | 5.6 | 83 | -4.8 | 7.6 | 66 | -5.7 | 6 | 24 | -5.7 | 6.0 | 24 | -1.6 | 5 | 52 | -3 | 5.6 | 83 | -4.4 | 5.7 | 9 |
| HCC | -3.7 | 6.3 | 75 | -5.5 | 7.2 | 51 | -7.6 | 6.1 | 18 | -7.6 | 6.1 | 18 | -1.8 | 3.8 | 47 | -3.7 | 6.3 | 75 | -8.0 | 5.3 | 8 |

Table 11: Percent of patient with at least 50% reduction in IEF (including traces) from baseline to 12 months post treatment according to treatment groups (PBO, LCC, HCC) and patient populations.

| | ITT | TPP1 | TPP2 | TPP3 | NR1 | NR2 | TPP3_damage |
|---|---|---|---|---|---|---|---|
| PBO | 40.3 | 38.6 | 31.6 | 34.8 | 46.7 | 43.8 | 25.0 |
| LCC | 47.6 | 48.9 | 45.5 | 50.0 | 35.3 | 46.2 | 55.6 |
| HCC | 54.1 | 57.9 | 56.8 | 61.1 | 41.7 | 55.3 | 75.0 |

Table 12: Percent of patient with at least 50% reduction in IEF (excluding traces) from baseline to 12 months post treatment according to treatment groups (PBO, LCC, HCC) and patient populations.

| | ITT | TPP1 | TPP3 | NR1 | NR2 | TPP3_DAMAGE |
|---|---|---|---|---|---|---|
| PBO | 43.2 | 38.7 | 30.4 | 46.2 | 53.2 | 25.0 |
| LCC | 56.4 | 50.8 | 62.5 | 56.3 | 53.1 | 66.7 |
| HCC | 66.2 | 60.0 | 72.2 | 56.5 | 64.4 | 87.5 |

*Table 13: Overview of patient populations analyzed according to Example 7 by number of patients (N), fecal incontinence severity, time since diagnosis of fecal incontinence and conditions associated with FI. M.D. = Muscle damage, P.F.D = Pelvic floor dysfunction, N.D = Nerve damage, LOSC = Loss of storage capacity, AT = Atrophy.*

| | ITT (N=237) | NR1 (N=57) | NR2 (N=149) | TPP1 (N=132) | TPP2 (N=119) | TPP3 (N=65) | TPP3_damage (N=29) |
|---|---|---|---|---|---|---|---|
| **FI SEVERITY [weekly IEF]** | 0-63 | Not specified | ≤2* | Not specified | >6 | >2* | >2* |
| **Time since first diagnosis of FI [years]** | 0-46 | >10 | ≥0 | ≤10 | ≤10 | ≤10 | ≤10 |
| **Conditions associated with FI** | Comprising M.D., P.F.D,, N.D., LOSC or AT | Comprising M.D., P.F.D,, N.D., LOSC or AT | Comprising M.D., P.F.D,, N.D., LOSC or AT | Comprising M.D., P.F.D,, N.D., LOSC or AT | Comprising M.D., P.F.D,, N.D., LOSC or AT | Comprising M.D., P.F.D,, N.D., LOSC or AT | M.D. but not P.F.D., N.D., LOSC or AT |

* episodes classified as little or more but without traces

**References**

Bajpai, V. K., Mistriotis, P., Loh, Y.-H., Daley, G. Q., & Andreadis, S. T. (2012). Functional vascular smooth muscle cells derived from human induced pluripotent stem cells via mesenchymal stem cell intermediates. Cardiovascular Research, 96(3), 391-400.

Frudinger, A., Kolle, D., Schwaiger, W., Pfeifer, J., Paede, J., & Halligan, S. (2009). Muscle- derived cell injection to treat anal incontinence due to obstetric trauma. Gut, 59(01), 55-

Frudinger, A., Pfeifer, J., Paede, J., Kolovetsiou-Kreiner, V., Marksteiner, R., & Halligan, S. (2015). Autologous skeletal-muscle-derived cell injection for anal incontinence due to obstetric trauma: a 5-year follow-up of an initial study of 10 patients. Colorectal Disease: The Official Journal of the Association of Coloproctology of Great Britain and Ireland, 17(9), 794-801.

Hirai, H., Yang, B., Garcia-Barrio, M. T., Rom, O., Ma, P. X., Zhang, J., & Chen, Y. E. (2018). Direct Reprogramming of Fibroblasts Into Smooth Muscle-Like Cells With Defined Transcription Factors-Brief Report. Arteriosclerosis, Thrombosis, and Vascular Biology, 38(9), 2191-2197.

Incitti, T., Magli, A., Jenkins, A., Lin, K., Yamamoto, A., & Perlingeiro, R. C. R. (2020). Pluripotent stem cell-derived skeletal muscle fibers preferentially express myosin heavy- chain isoforms associated with slow and oxidative muscles. Skeletal Muscle, 10(1), 17.

Ito, N., Kii, I., Shimizu, N., Tanaka, H., & Takeda, S. (2017). Direct reprogramming of fibroblasts into skeletal muscle progenitor cells by transcription factors enriched in undifferentiated subpopulation of satellite cells. Scientific Reports, 7(1), 8097.

Jorge, J. Marcio N., Wexner, Steven D. (1993). Etiology and Management of Fecal Incontinence. Dis Colon Rectum, Vol. 36, No.1, pp. 77-96.

Messner, F., Thurner, M., Müller, J., Blumer, M., Hofmann, J., Marksteiner, R., Couillard-Despres, S., Troppmair, J., Ofner, D., Schneeberger, S., & Hautz, T. (2021). Myogenic progenitor cell transplantation for muscle regeneration following hindlimb ischemia and reperfusion. Stem Cell Research & Therapy, 12(1), 146.

Miyagoe-Suzuki, Y., & Takeda, S. (2017). Skeletal muscle generated from induced pluripotent stem cells - induction and application. World Journal of Stem Cells, 9(6), 89-97.

Rando, T. A., & Blau, H. M. (1994). Primary mouse myoblast purification, characterization, and transplantation for cell-mediated gene therapy. The Journal of Cell Biology, 125(6), 1275-1287.

Rao, S. S. C. (2016). Endpoints for therapeutic interventions in faecal incontinence: small step or game changer. In Neurogastroenterology and Motility (Vol. 28, Issue 8, pp. 1123-1133). Blackwell Publishing Ltd.

Takahashi, K., Tanabe, K., Ohnuki, M., Narita, M., Ichisaka, T., Tomoda, K., & Yamanaka, S. (2007). Induction of pluripotent stem cells from adult human fibroblasts by defined factors. Cell, 131(5), 861-872.

Takahashi, K., & Yamanaka, S. (2006). Induction of pluripotent stem cells from mouse embryonic and adult fibroblast cultures by defined factors. Cell, 126(4), 663-676.

Thurner, M., Deutsch, M., Janke, K., Messner, F., Kreutzer, C., Beyl, S., Couillard-Després, S., Hering, S., Troppmair, J., & Marksteiner, R. (2020). Generation of myogenic progenitor cell-derived smooth muscle cells for sphincter regeneration. Stem Cell Research & Therapy, 11(1), 233.

Xuan, W., Khan, M., & Ashraf, M. (2021). Pluripotent stem cell-induced skeletal muscle progenitor cells with givinostat promote myoangiogenesis and restore dystrophin in injured Duchenne dystrophic muscle. Stem Cell Research & Therapy, 12(1), 131.

Yamanaka, S. (2008). Induction of pluripotent stem cells from mouse fibroblasts by four transcription factors. Cell Proliferation, 41 Suppl 1, 51-56.

**Claims**

1. Myoblasts for use in a method for the treatment of anal incontinence in a subject, wherein the myoblasts are administered by one or more injections into and/or adjacent to the anal sphincter apparatus of said subject, wherein said subject suffers from anal incontinence for 6 months to 10 years and the subject has a severity of incontinence defined as more than 2 weekly incontinence episodes prior to treatment, wherein said incontinence episodes result in that liquid or solid feces unexpectedly leaked from the rectum of a subject, and wherein incontinence episodes that only appear as traces in form of staining or marks in the linen are excluded.

2. The myoblasts for use according to claim 1, wherein said incontinence episodes result in that the amount of feces is more than appearing as staining or marks in the linen of the subject and its volume is comparable to what a normal defecation would result in.

3. The myoblasts for use according to claim 1 or 2, wherein the subject's anal incontinence is caused by muscle damage.

4. The myoblasts for use according to any one of claims 1 to 3, wherein the subject has a severity of incontinence defined as more than 6, preferably more than 7, preferably more than 8, preferably more than 9 or preferably more than 10 weekly incontinence episodes prior to treatment.

5. The myoblasts for use according to any one of claims 1 to 4, wherein the method comprises stimulation of the anal sphincter apparatus prior to and/or after administration of myoblasts.

6. The myoblasts for use according to claim 5, wherein stimulation comprises at least 2 weeks of anal sphincter apparatus stimulation after administration of myoblasts, more preferably at least 2 weeks of anal sphincter apparatus stimulation prior to and after administration of myoblasts.

7. The myoblasts for use according to claim 5 or 6, wherein the stimulation is performed by Kegel exercise and/or by percutaneous electrical stimulation.

8. The myoblasts for use according to claim 7, wherein the percutaneous electrical stimulation is performed daily and preferably at least twice or at least trice per day.

9. The myoblasts for use according to any one of claim 5 to 8, wherein each stimulation is for at least 10 minutes, more preferably for at least 20 minutes.

10. The myoblasts for use according to any one of claims 1 to 9, wherein the myoblasts are **characterized**

   (i) **by** a positive expression of the markers of CD56 and CD90, in particular wherein at least 60 % of the myoblasts express CD56 and at least 60 % of the myoblasts express CD90, and/or
   (ii) by an AChE activity of about 20 $mU_{rel}$ to about 1000 $mU_{rel}$, more preferably of about 30 $mU_{rel}$ to about 800 $mU_{rel}$, more preferably about 50 to about 700 $mU_{rel}$, wherein said AChE activity is determined per $2*10^5$ cells.

11. The myoblasts for use according to any one of claims 1 to 10, wherein about 60 to about 99.9% of the myoblasts express the cell marker CD56, about 80 to about 99.9% of the myoblasts express the cell marker CD90 and about 0 to about 10% of the myoblasts express the cell marker CD34.

12. The myoblasts for use according to any one of claims 1 to 11, wherein the method comprises the administration of an effective amount of myoblasts, preferably

   (i) the administration of about 1 to about 200 million myoblasts, about 4 to about 60 million myoblasts, about 4 to about 6 million myoblasts or about 40 to about 60 million myoblasts, and/or
   (ii) the administration of an amount of myoblasts having a total AChE activity of about $1*10^2$ $mU_{rel\_total}$ to about $1*10^6$ $mU_{rel\_total}$, more preferably of about $1*10^3$ $mU_{rel\_total}$ to about $5*10^5$ $mU_{rel\_total}$ and even more preferably of about $7*10^4$ $mU_{rel\_total}$ to about $2*10^5$ $mU_{rel\_total}$.

13. The myoblasts for use according to any one of claims 1 to 12, wherein the myoblasts are administered by one or more injections into the external anal sphincter muscle, the internal anal sphincter muscle and/or into the puborectalis muscle.

14. The myoblasts for use according to any one of claims 1 to 13, wherein the myoblasts are comprised in a pharmaceutical composition comprising a pharmaceutical acceptable excipient and/or carrier.

**Patentansprüche**

1. Myoblasten zur Verwendung in einem Verfahren zur Behandlung von Stuhlinkontinenz bei einem Patienten, wobei die Myoblasten durch eine oder mehrere Injektionen in und/oder neben den Analsphinkterapparat des Patienten verabreicht werden, wobei der Patient seit 6 Monaten bis 10 Jahren an Stuhlinkontinenz leidet und der Patient eine Schwere der Inkontinenz aufweist, die definiert ist als mehr als 2 wöchentliche Inkontinenzepisoden vor der Behandlung, wobei diese Inkontinenzepisoden dazu führen, dass flüssige oder feste Fäzes unerwartet aus dem Rektum des Patienten austreten, und wobei solche Inkontinenzepisoden ausgeschlossen sind, die sich lediglich als Spuren in Form von Verfärbungen oder Flecken in der Wäsche äußern.

2. Die Myoblasten zur Verwendung nach Anspruch 1, wobei die Inkontinenzepisoden dazu führen, dass die Menge an Fäzes mehr als nur Flecken oder Spuren in der Wäsche des Patienten hinterlässt und deren Volumen mit dem einer normalen Darmentleerung vergleichbar ist.

3. Die Myoblasten zur Verwendung nach Anspruch 1 oder 2, wobei die Stuhlinkontinenz des Patienten durch eine Muskelschädigung verursacht ist.

4. Die Myoblasten zur Verwendung nach einem der Ansprüche 1 bis 3, wobei der Patient vor der Behandlung einen Schweregrad der Inkontinenz aufweist, der als mehr als 6, vorzugsweise mehr als 7, vorzugsweise mehr als 8, vorzugsweise mehr als 9 oder vorzugsweise mehr als 10 wöchentliche Inkontinenzepisoden definiert ist.

5. Die Myoblasten zur Verwendung nach einem der Ansprüche 1 bis 4, wobei das Verfahren die Stimulation des Analsphinkters vor und/oder nach der Verabreichung der Myoblasten umfasst.

6. Die Myoblasten zur Verwendung nach Anspruch 5, wobei die Stimulation mindestens zwei Wochen Stimulation des Analsphinkterapparats nach Verabreichung der Myoblasten umfasst, vorzugsweise mindestens zwei Wochen Stimulation des Analsphinkterapparats vor und nach Verabreichung der Myoblasten.

7. Die Myoblasten zur Verwendung nach Anspruch 5 oder 6, wobei die Stimulation durch Kegel-Übungen und/oder durch perkutane elektrische Stimulation durchgeführt wird.

8. Die Myoblasten zur Verwendung nach Anspruch 7, wobei die perkutane elektrische Stimulation täglich und vorzugsweise mindestens zweimal oder mindestens dreimal pro Tag durchgeführt wird.

9. Die Myoblasten zur Verwendung nach einem der Ansprüche 5 bis 8, wobei jede Stimulation mindestens 10 Minuten, vorzugsweise mindestens 20 Minuten dauert.

10. Die Myoblasten zur Verwendung nach einem der Ansprüche 1 bis 9, wobei die Myoblasten **gekennzeichnet sind**

(i) **durch** eine positive Expression der Marker CD56 und CD90, insbesondere wobei mindestens 60 % der Myoblasten CD56 exprimieren und mindestens 60 % der Myoblasten CD90 exprimieren, und/oder
(ii) **durch** eine AChE-Aktivität von etwa 20 $mU_{rel}$ bis etwa 1000 $mU_{rel}$, vorzugsweise von etwa 30 $mU_{rel}$ bis etwa 800 $mU_{rel}$, noch bevorzugter von etwa 50 bis etwa 700 $mU_{rel}$, wobei die AChE-Aktivität pro $2*10^5$ Zellen bestimmt wird.

11. Die Myoblasten zur Verwendung nach einem der Ansprüche 1 bis 10, wobei etwa 60 bis etwa 99,9 % der Myoblasten den Zellmarker CD56 exprimieren, etwa 80 bis etwa 99,9 % der Myoblasten den Zellmarker CD90 exprimieren und etwa 0 bis etwa 10 % der Myoblasten den Zellmarker CD34 exprimieren.

12. Die Myoblasten zur Verwendung nach einem der Ansprüche 1 bis 11, wobei das Verfahren die Verabreichung einer wirksamen Menge an Myoblasten umfasst, vorzugsweise

(i) die Verabreichung von etwa 1 bis etwa 200 Millionen Myoblasten, etwa 4 bis etwa 60 Millionen Myoblasten, etwa 4 bis etwa 6 Millionen Myoblasten oder etwa 40 bis etwa 60 Millionen Myoblasten, und/oder
(ii) die Verabreichung einer Menge an Myoblasten mit einer Gesamt-AChE-Aktivität von etwa $1*10^2$ $mU_{rel\_total}$ bis etwa $1*10^6$ $mU_{rel\_total}$, vorzugsweise von etwa $1*10^3$ $mU_{rel\_total}$ bis etwa $5*10^5$ $mU_{rel\_total}$ und noch bevorzugter von etwa $7*10^4$ $mU_{rel\_total}$ bis etwa $2*10^5$ $mU_{rel\_total}$.

13. Die Myoblasten zur Verwendung nach einem der Ansprüche 1 bis 12, wobei die Myoblasten durch eine oder mehrere Injektionen in den äußeren Analsphinktermuskel, den inneren Analsphinktermuskel und/oder in den Musculus puborectalis verabreicht werden.

14. Die Myoblasten zur Verwendung nach einem der Ansprüche 1 bis 13, wobei die Myoblasten in einer pharmazeutischen Zusammensetzung enthalten sind, die einen pharmazeutisch verträglichen Hilfsstoff und/oder Träger umfasst.

**Revendications**

1.  Myoblastes destinés à être utilisés dans une méthode de traitement de l'incontinence anale chez un sujet, où les myoblastes sont administrés par une ou plusieurs injections dans et/ou à un endroit adjacent à l'appareil de sphincter anal dudit sujet, où ledit sujet est atteint d'incontinence anale depuis 6 mois à 10 ans et le sujet a une gravité d'incontinence définie comme étant supérieure à 2 épisodes d'incontinence par semaine avant le traitement, où lesdits épisodes d'incontinence aboutissent à ce que des fèces liquides ou solides fuient de manière inattendue par le rectum d'un sujet, et où les épisodes d'incontinence qui apparaissent seulement en tant que traces sous la forme de coloration ou marques dans le linge sont exclus.

2.  Myoblastes destinés à être utilisés selon la revendication 1, où lesdits épisodes d'incontinence aboutissent à ce que la quantité de fèces fait plus qu'apparaître sous la forme de coloration ou marques dans le linge du sujet et leur volume est comparable à ce qu'une défécation normale donnerait.

3.  Myoblastes destinés à être utilisés selon la revendication 1 ou 2, où l'incontinence anale du sujet est provoquée par une lésion musculaire.

4.  Myoblastes destinés à être utilisés selon l'une quelconque des revendications 1 à 3, où le sujet connaît une gravité d'incontinence définie comme étant supérieure à 6, de préférence supérieure à 7, de préférence supérieure à 8, de préférence supérieure à 9 ou de préférence supérieure à 10 épisodes d'incontinence par semaine avant le traitement.

5.  Myoblastes destinés à être utilisés selon l'une quelconque des revendications 1 à 4, où la méthode comprend la stimulation de l'appareil de sphincter anal avant et/ou après l'administration des myoblastes.

6.  Myoblastes destinés à être utilisés selon la revendication 5, où la stimulation comprend au moins 2 semaines de stimulation de l'appareil de sphincter anal après l'administration des myoblastes, de manière préférée au moins 2 semaines de stimulation de l'appareil de sphincter anal avant et après l'administration des myoblastes.

7.  Myoblastes destinés à être utilisés selon la revendication 5 ou 6, où la stimulation est réalisée par un exercice de Kegel et/ou par stimulation électrique percutanée.

8.  Myoblastes destinés à être utilisés selon la revendication 7, où la stimulation électrique percutanée est réalisée quotidiennement et de préférence au moins deux fois ou au moins trois fois par jour.

9.  Myoblastes destinés à être utilisés selon l'une quelconque des revendications 5 à 8, où chaque stimulation dure au moins 10 minutes, de manière préférée dure au moins 20 minutes.

10. Myoblastes destinés à être utilisés selon l'une quelconque des revendications 1 à 9, où les myoblastes sont **caractérisés**

    (i) **par** une expression positive des marqueurs de CD56 et CD90, en particulier où au moins 60 % des myoblastes expriment CD56 et au moins 60 % des myoblastes expriment CD90 ; et/ou
    (ii) par une activité AChE d'environ 20 $mU_{rel}$ à environ 1000 $mU_{rel}$, de manière préférée d'environ 30 $mU_{rel}$ à environ 800 $mU_{rel}$, de manière encore préférée d'environ 50 à environ 700 $mU_{rel}$, où ladite activité AChE est déterminée pour $2*10^5$ cellules.

11. Myoblastes destinés à être utilisés selon l'une quelconque des revendications 1 à 10, où environ 60 à environ 99,9 % des myoblastes expriment le marqueur cellulaire CD56, environ 80 à 99,9 % des myoblastes expriment le marqueur cellulaire CD90 et environ 0 à 10 % des myoblastes expriment le marqueur cellulaire CD34.

12. Myoblastes destinés à être utilisés selon l'une quelconque des revendications 1 à 11, où la méthode comprend l'administration d'une quantité efficace de myoblastes, de préférence

    (i) l'administration d'environ 1 à environ 200 millions de myoblastes, d'environ 4 à environ 60 millions de myoblastes, d'environ 4 à environ 6 millions de myoblastes ou d'environ 40 à environ 60 millions de myoblastes ; et/ou
    (ii) l'administration d'une quantité de myoblastes ayant une activité AChE totale d'environ $1*10^2$ $mU_{rel\_total}$ à environ $1*10^6$ $mU_{rel\_total}$, de manière préférée d'environ $1*10^3$ $mU_{rel\_total}$ à environ $5*10^5$ $mU_{rel\_total}$ et même de manière encore préférée d'environ $7*10^4$ mU$_{rel\_total}$ à environ $2*10^5$ mU$_{rel\_total}$.

13. Myoblastes destinés à être utilisés selon l'une quelconque des revendications 1 à 12, où les myoblastes sont administrés par une ou plusieurs injections dans le muscle du sphincter anal externe, le muscle du sphincter anal interne, et/ou dans le muscle puborectal.

14. Myoblastes destinés à être utilisés selon l'une quelconque des revendications 1 à 13, où les myoblastes sont compris dans une composition pharmaceutique comprenant un excipient et/ou un véhicule pharmaceutique acceptable.

Figure 1

**A**

IEF_Time_course_ITT

*p=0.035

**B**

IEF_Time_course_ITT_WT

*p=0.034

**C**

Responder-rate_Time_course_ITT

**D**

Responder-rate_Time_course_ITT_wt

§ p=0.037
* p=0.006

EP 4 380 583 B1

# Figure 2

**A** Change in IEF

$\Delta$ IEF (V0 to V5)

Treatment arm: PBO, LCC, HCC

NR1, NR2, ITT, TPP1, TPP2, TPP3

* p=0.015
& p=0.033
%, # p<0.05

**B** Change in IEF (traces not counted)

$\Delta$ IEF (V0 to V5)

Treatment arm: PBO, LCC, HCC

NR1, NR2, ITT, TPP1, TPP3

* p=0.008
§ p=0.015
& p=0.043
# p=0.034

**C** Responder rates

Responder rate 50% at V5

Treatment arm: PBO, LCC, HCC

NR1, NR2, ITT, TPP1, TPP2, TPP3

* p=0.028
& p=0.033

**D** Responder rates (traces not counted)

Responder rate 50% at V5

Treatment arm: PBO, LCC, HCC

NR2, NR1, ITT, TPP1, TPP3

* p= 0.008
$ p= 0.006
§ p= 0.028
& p= 0.025

Figure 3

# Figure 4

**A**

| | |
|---|---|
| V-2 :Screening | △ ◣ ⬤ ✦ ▲ ✛ |
| V2: 7 days post implantation | ◤ ✛ |
| V3: 3 Months post implantation | ◎ △ ◇ ◣ ✛ |
| V4: 6 Months post implantation | ◎ △ ◇ ⬤ ◣ ✦ ✛ ◼ ▲ |

Baseline

| | |
|---|---|
| V-1: Biopsy | ◎ ◇ ✦ |
| V0/V1:Implantation | ◎ △ ◇ ◣ ◼ ◤ ✛ |
| V5: 12 Months post implantation | ◎ ◇ ◣ ✦ |

Legend:
- ▢ Diary (1 week)
- ⬠ Electrical stimulation (1 week)
- ◎ Diary Collection (WIE)
- △ Wexner Score
- ◇ VAS
- ⬤ Anorectal Manometry
- ◣ QoL Questionnaire
- ✦ CGI
- ◼ 3D Sonography
- ▲ Rectoscopy and ultrasound
- ◤ Post implantation control
- ✛ Safety analysis

**B**

Screened N=288 → Randomized N=251 → Intention-to-treat N=237 → Visit 5 performed N=235

Screening failure N=3
- Not eligible for inclusion (N=28)
- Withdrawal of consent (N=8)
- Lost to follow-up (N=1)

Allocated to PBO N=79

Allocated to LCC N=83

Allocated to HCC N=75

EP 4 380 583 B1

Figure 5

Figure 6

A

B

Figure 7

# Figure 8

EP 4 380 583 B1

# Figure 9

**A**

Change in IEF

**B**

Change in IEF (traces not counted)

**C**

Responder rates

**D**

Responder rates
(traces not counted)

EP 4 380 583 B1

# Figure 10

**A**

Effect sizes of IEF changes

**B**

Effect sizes of IEF changes
(traces not counted)

**C**

Odds ratios of Responder rates

**D**

Odds ratios of Responder rates
(traces not counted)

EP 4 380 583 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 210976 B1
- WO 2014044867 A1
- WO 2019115790 A
- WO 2020193460 A
- EP 2120976 B1
- US 5538722 A

### Non-patent literature cited in the description

- **THURNER et al.** *Stem Cell Research & Therapy*, vol. 11 (1), 2020
- **ROMANISZYN et al.** Techniques in Coloproctology. Springer-Verlag Italia, 2015, vol. 19, 685-696
- **FRUDINGER et al.** *Colorectal Disease*, 2015, vol. 17 (9), 794-801
- **WEBSTER et al.** *Exp Cell Res.*, January 1988, vol. 174 (1), 252-65
- **BAJPAI, V. K.** ; **MISTRIOTIS, P.** ; **LOH, Y.-H.** ; **DALEY, G. Q.** ; **ANDREADIS, S. T.** Functional vascular smooth muscle cells derived from human induced pluripotent stem cells via mesenchymal stem cell intermediates.. *Cardiovascular Research*, 2012, vol. 96 (3), 391-400
- **FRUDINGER, A.** ; **KOLLE, D.** ; **SCHWAIGER, W.** ; **PFEIFER, J.** ; **PAEDE, J.** ; **HALLIGAN, S.** Muscle-derived cell injection to treat anal incontinence due to obstetric trauma.. *Gut*, 2009, vol. 59 (01), 55
- **FRUDINGER, A.** ; **PFEIFER, J.** ; **PAEDE, J.** ; **KOLOVETSIOU-KREINER, V.** ; **MARKSTEINER, R.** ; **HALLIGAN, S.** Autologous skeletal-muscle-derived cell injection for anal incontinence due to obstetric trauma: a 5-year follow-up of an initial study of 10 patients.. *Colorectal Disease: The Official Journal of the Association of Coloproctology of Great Britain and Ireland*, 2015, vol. 17 (9), 794-801
- **HIRAI, H.** ; **YANG, B.** ; **GARCIA-BARRIO, M. T.** ; **ROM, O.** ; **MA, P. X.** ; **ZHANG, J.** ; **CHEN, Y. E.** Direct Reprogramming of Fibroblasts Into Smooth Muscle-Like Cells With Defined Transcription Factors-Brief Report.. *Arteriosclerosis, Thrombosis, and Vascular Biology*, 2018, vol. 38 (9), 2191-2197
- **INCITTI, T.** ; **MAGLI, A.** ; **JENKINS, A.** ; **LIN, K.** ; **YAMAMOTO, A.** ; **PERLINGEIRO, R. C. R.** Pluripotent stem cell-derived skeletal muscle fibers preferentially express myosin heavy- chain isoforms associated with slow and oxidative muscles.. *Skeletal Muscle*, 2020, vol. 10 (1), 17

- **ITO, N.** ; **KII, I.** ; **SHIMIZU, N.** ; **TANAKA, H.** ; **TAKEDA, S.** Direct reprogramming of fibroblasts into skeletal muscle progenitor cells by transcription factors enriched in undifferentiated subpopulation of satellite cells.. *Scientific Reports*, 2017, vol. 7 (1), 8097
- **JORGE, J.** ; **MARCIO N.** ; **WEXNER, STEVEN D.** Etiology and Management of Fecal Incontinence. *Dis Colon Rectum*, 1993, vol. 36 (1), 77-96
- **MESSNER, F.** ; **THURNER, M.** ; **MÜLLER, J.** ; **BLUMER, M.** ; **HOFMANN, J.** ; **MARKSTEINER, R.** ; **COUILLARD-DESPRES, S.** ; **TROPPMAIR, J.** ; **OFNER, D.** ; **SCHNEEBERGER, S.** Myogenic progenitor cell transplantation for muscle regeneration following hindlimb ischemia and reperfusion.. *Stem Cell Research & Therapy*, 2021, vol. 12 (1), 146
- **MIYAGOE-SUZUKI, Y.** ; **TAKEDA, S.** Skeletal muscle generated from induced pluripotent stem cells - induction and application. *World Journal of Stem Cells*, 2017, vol. 9 (6), 89-97
- **RANDO, T. A.** ; **BLAU, H. M.** Primary mouse myoblast purification, characterization, and transplantation for cell-mediated gene therapy.. *The Journal of Cell Biology*, 1994, vol. 125 (6), 1275-1287
- Endpoints for therapeutic interventions in faecal incontinence: small step or game changer. **RAO, S. S. C.** Neurogastroenterology and Motility. Blackwell Publishing Ltd, 2016, vol. 28, 1123-1133
- **TAKAHASHI, K.** ; **TANABE, K.** ; **OHNUKI, M.** ; **NARITA, M.** ; **ICHISAKA, T.** ; **TOMODA, K.** ; **YAMANAKA, S.** Induction of pluripotent stem cells from adult human fibroblasts by defined factors.. *Cell*, 2007, vol. 131 (5), 861-872
- **TAKAHASHI, K.** ; **YAMANAKA, S.** Induction of pluripotent stem cells from mouse embryonic and adult fibroblast cultures by defined factors.. *Cell*, 2006, vol. 126 (4), 663-676

- **THURNER, M.** ; **DEUTSCH, M.** ; **JANKE, K.** ; **MESSNER, F.** ; **KREUTZER, C.** ; **BEYL, S.** ; **COUILLARD-DESPRÉS, S.** ; **HERING, S.** ; **TROPP-MAIR, J.** ; **MARKSTEINER, R.** Generation of myogenic progenitor cell-derived smooth muscle cells for sphincter regeneration.. *Stem Cell Research & Therapy*, 2020, vol. 11 (1), 233

- **XUAN, W.** ; **KHAN, M.** ; **ASHRAF, M.** Pluripotent stem cell-induced skeletal muscle progenitor cells with givinostat promote myoangiogenesis and restore dystrophin in injured Duchenne dystrophic muscle.. *Stem Cell Research & Therapy*, 2021, vol. 12 (1), 131
- **YAMANAKA, S.** Induction of pluripotent stem cells from mouse fibroblasts by four transcription factors.. *Cell Proliferation*, 2008, vol. 41 (1), 51-56